# EUROPEAN PATENT SPECIFICATION

(11) **EP 0 565 377 B1**
(45) Date of publication and mention of the grant of the patent: **07.01.1998**
(21) Application number: 93302780.7
(22) Date of filing: 08.04.1993
(51) Int. Cl.: A61K 31/52, C07D 473/06

(54) **Therapeutic agents for use in the treatment of Parkinson's Disease**
Arzneimittel zur Verwendung bei der Behandlung von Parkinsonismus
Médicaments pour l'emploi dans le traitement de la maladie de Parkinson

(30) Priority: 08.04.1992 JP 87115/92
(43) Date of publication of application: 13.10.1993
(73) Proprietor: Kyowa Hakko Kogyo Co., Ltd., Chiyoda-ku, Tokyo 100 (JP)
(72) Inventor: Suzuki, Fumio, Mishima-shi, Shizuoka-ken (JP); Shimada, Junichi, Sunto-gun, Shizuoka-ken (JP); Ishii, Akio, Sunto-gun, Shizuoka-ken (JP); Ichikawa, Shunji, Tagata-gun, Shizuoka-ken (JP)
(74) Representative: Harding, Charles Thomas

(56) References cited:
- EP-A- 0 374 808
- EP-A- 0 389 282
- EP-A- 0 470 317
- WO-A-92/00297
- WO-A-92/06976
- NL-A- 7 011 094
- J.MED.CHEM vol. 34, 1991, pages 1431 - 5
- CHEM.BER. vol. 119, 1986, pages 1525 - 39

## Description

The present invention relates to various xanthine derivatives and salts thereof now found to be useful in the treatment of Parkinson's disease.

Various derivatives of xanthine are known to have pharmacological activity, for example, compounds of formulae A and B:

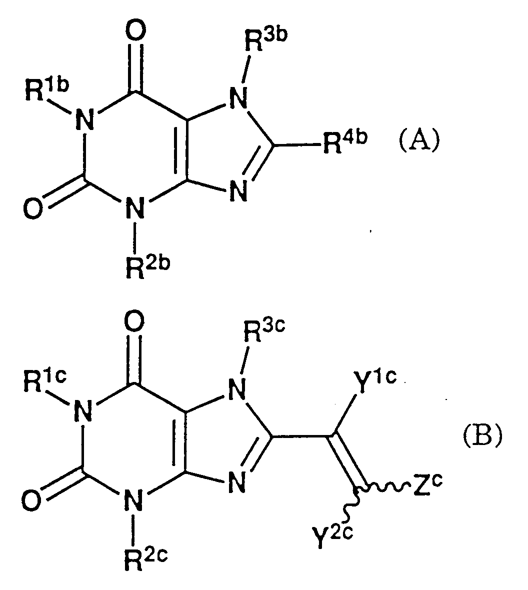

Compounds of Formula (A), for example, in which R^{1b} and R^{2b} both represent propyl, R^{3b} represents hydrogen, and R^{4b} represents substituted or unsubstituted phenyl, aromatic heterocyclic group, cycloalkyl, styryl, or phenylethyl are known to be adenosine antagonists [J. Med. Chem., 34, 1431 (1991)], whilst compounds of Formula (B) in which R^{1c} and R^{2c} independently represent methyl or ethyl, R^{3c} represents methyl, Y^{1c} and Y^{2c} represent hydrogen, and Z^{c} represents phenyl or 3,4,5-trimethoxyphenyl are known stimulants of brain activity [JP-A-26516/72].

Compounds of Formula (B) in which R^{1c} and R^{2c} independently represent hydrogen, propyl, butyl, or allyl, R^{3c} represents hydrogen or lower alkyl, Y^{1c} and Y^{2c} independently represent hydrogen or methyl, and Z^{c} represents phenyl, pyridyl, imidazolyl, furyl, or thienyl unsubstituted or substituted by 1 to 3 substituents such as lower alkyl, hydroxy, lower alkoxy, halogen, amino, and nitro are also known to be adenosine A₂ receptor antagonists [WO 92/06976]. Other compounds of Formula (B) are also known, but without any indication as to their pharmacological action, if any. For example, 8-styryl caffeine, which is a compound of Formula (B) in which R^{1c}, R^{2c}, and R^{3c} represent methyl, Y^{1c} and Y^{2c} represent hydrogen, and Z^{c} represents phenyl, is disclosed in Chem. Ber. 119, 1525 (1986) whilst the compound of Formula (B), in which R^{1c}, R^{2c}, and R^{3c} represent methyl, Y^{1c} and Y^{2c} represent hydrogen, and Z^{c} represents pyridyl, quinolyl, or methoxy-substituted or unsubstituted benzothiazolyl is disclosed in Chem. Abst. 60, 1741h (1964).

In addition to the foregoing, xanthine derivatives of formula A have also been suggested as having utility in the treatment of Parkinson's disease, see EP-A-0374808, WO92/00297 and EP-A-0389282.

In accordance with the present invention a group of xanthine compounds has been discovered showing excellent therapeutic properties in the treatment of Parkinson's disease. These are xanthine derivatives of the Formula (I): 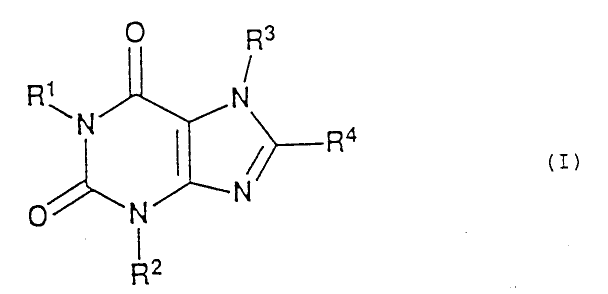 in which R¹, R², and R³ independently represent hydrogen, lower alkyl, or allyl; and R⁴ represents a 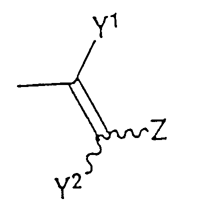 group, where Y¹ and Y² independently represent hydrogen or methyl; and Z represents substituted or unsubstituted aryl or a substituted or unsubstituted heterocyclic group, and their pharmaceutically acceptable salts.

The compounds represented by Formula (I) are hereinafter referred to as Compounds (I), and the same applies to the compounds of other formula numbers.

In the definitions of the groups in Formula (I), the lower alkyl means a straight-chain or branched alkyl group having 1 to 6 carbon atoms, such as methyl, ethyl, propyl, isopropyl, butyl, isobutyl, sec-butyl, tert-butyl, pentyl, neopentyl, and hexyl. The aryl means an aryl group having 6 to 10 carbon atoms, such as phenyl and naphthyl. Examples of the heterocyclic group are furyl, thienyl, pyrrolyl, pyranyl, thiopyranyl, pyridyl, thiazolyl, imidazolyl, pyrimidyl, triazinyl, indolyl, quinolyl, purinyl, and benzothiazolyl. The substituted aryl, such as the substituted naphthyl, and the substituted heterocyclic ring each has 1 to 3 independently-selected substituents. Examples of the substituents are lower alkyl, hydroxy, lower alkoxy, halogen, nitro, and amino. The lower alkyl and the alkyl moiety of the lower alkoxy have the same meaning as the lower alkyl defined above. The halogen includes fluorine, chlorine, bromine, and iodine.

The above-mentioned pharmaceutically acceptable salts of Compounds (I) include pharmaceutically acceptable acid addition salts, metal salts, ammonium salts, organic amine addition salts, and amino acid addition salts.

Examples of the pharmaceutically acceptable acid addition salts are inorganic acid addition salts such as hydrochloride, sulfate, and phosphate, and organic acid addition salts such as acetate, maleate, fumarate, tartrate, and citrate. Examples of the pharmaceutically acceptable metal salts are alkali metal salts such as sodium salt and potassium salt, alkaline earth metal salts such as magnesium salt and calcium salt, aluminium salt, and zinc salt. Examples of the pharmaceutically acceptable ammonium salts are ammonium salt and tetramethyl ammonium salt. Examples of the pharmaceutically acceptable organic amine addition salts are salts with morpholine and piperidine. Examples of the pharmaceutically acceptable amino acid addition salts are salts with lysine, glycine, and phenylalanine.

The processes for producing Compounds (I) are described below. Compounds (I) can also be produced according to the methods described in, for example, Japanese Published Unexamined Patent Application No. 26516/72; J. Med. Chem., 34, 1431 (1991); Chem. Ber., 119, 1525 (1986); and Chem. Abst., 60, 1741h (1964).

### Process 1

Compound (I-b) [Compound (I) in which R³ is hydrogen] can be prepared by the following reaction steps:

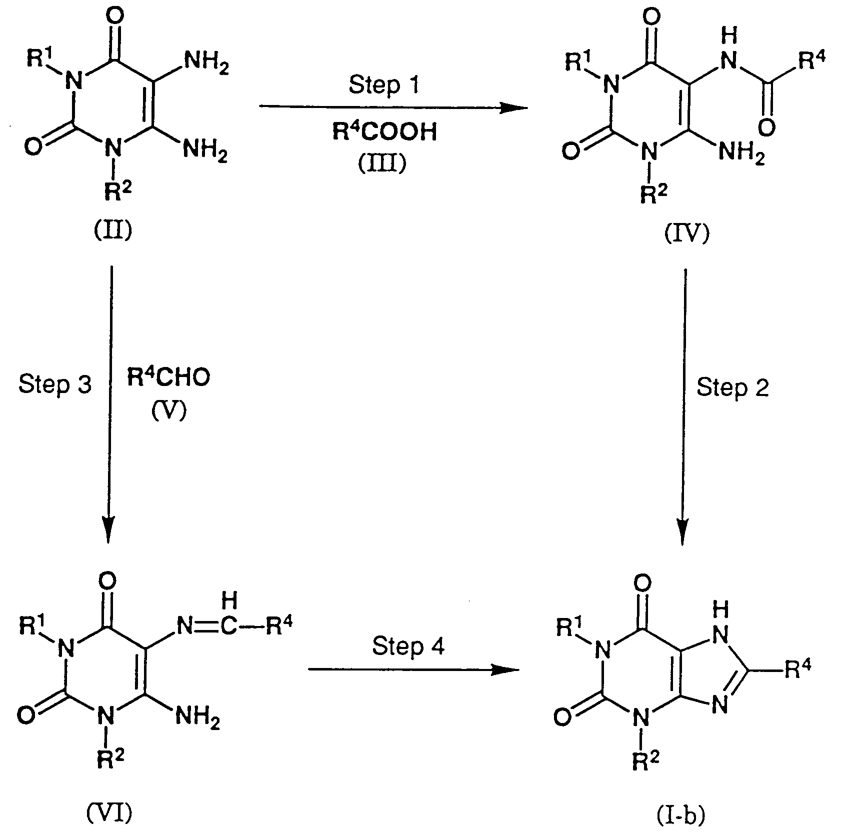

(In the formulae, R¹, R², and R⁴ have the same meanings as defined above.)

### (STEP 1)

A uracil derivative (II) obtained by a known method (for example, Japanese Published Unexamined Patent Application No. 42383/84) is allowed to react with either a carboxylic acid (III) or a reactive derivative thereof to give Compound (IV). Examples of the reactive derivative of the carboxylic acid (III) are acid halides such as acid chloride and acid bromide, active esters such as p-nitrophenyl ester and N-oxysuccinimide, commercially available acid anhydrides, acid anhydrides produced by using carbodiimides such as 1-ethyl-3-(3-dimethylaminopropyl)carbodiimide, diisopropyl carbodiimide and dicyclohexyl carbodiimide, and mixed acid anhydrides with monoethyl carbonate or monoisobutyl carbonate. If the carboxylic acid (III) is used, the reaction is completed in 10 minutes to 5 hours at 50 to 200°C without using a solvent.

If a reactive derivative of the carboxylic acid (III) is used, the reaction can be carried out according to a conventional method employed in peptide chemistry. That is, Compound (II) and a derivative of the carboxylic acid (III) are allowed to react in a solvent, preferably in the presence of an additive or a base, to give Compound (IV). Examples of the solvent are halogenated hydrocarbons such as methylene chloride, chloroform, and ethylene dichloride, ethers such as dioxane and tetrahydrofuran, dimethylformamide, dimethylsulfoxide, and water. An example of the additive is 1-hydroxybenzotriazole. Examples of the base are pyridine, triethylamine, 4-dimethylaminopyridine, and N-methylmorpholine. The reaction is completed in 0.5 to 24 hours at -80 to 50°C. The reactive derivative may be formed in the reaction system and then used without being isolated.

### (STEP 2)

Compound (I-b) can be obtained by reaction of Compound (IV) carried out in any of the following manners: in the presence of a base (Method A); by treatment with a dehydrating agent (Method B); or by heating (Method C). In Method A, the reaction is carried out in a solvent in the presence of a base such as an alkali metal hydroxide (e.g. sodium hydroxide and potassium hydroxide). As the solvent, water, lower alcohols such as methanol and ethanol, ethers such as dioxane and tetrahydrofuran, dimethylformamide, dimethylsulfoxide, and the like may be used alone or in combination. The reaction is completed in 10 minutes to 6 hours at 0 to 180°C.

In Method B, the reaction is carried out in an inert solvent or in the absence of a solvent using a dehydrating agent such as a thionyl halide (e.g. thionyl chloride) and a phosphorus oxyhalide (e.g. phosphorus oxychloride). Examples of the inert solvent are halogenated hydrocarbons such as methylene chloride, chloroform and ethane dichloride, dimethylformamide, and dimethylsulfoxide. The reaction is completed in 0.5 to 12 hours at 0 to 180°C.

In Method C, the reaction is carried out in a polar solvent such as dimethylformamide, dimethylsulfoxide, and Dowtherm A (Dow Chemicals). The reaction is completed in 10 minutes to 5 hours at 50 to 200°C.

### (STEP 3)

Compound (II) is allowed to react with an aldehyde (V) to give a Schiff's base (VI). As a reaction solvent, mixtures of acetic acid and a lower alcohol such as methanol or ethanol may be used. The reaction is completed in 0.5 to 12 hours at -20 to 100°C.

### (STEP 4)

Compound (VI) is oxidatively cyclized in an inert solvent in the presence of an oxidizing agent to form Compound (I-b). Examples of the oxidizing agent are oxygen, ferric chloride, cerium ammonium nitrate, and diethylazodicarboxylate. Examples of the inert solvent are lower alcohols such as methanol and ethanol, halogenated hydrocarbons such as methylene chloride and chloroform, and aromatic hydrocarbons such as toluene, xylene, and nitrobenzene. The reaction is completed in 10 minutes to 12 hours at 0 to 180°C.

### Process 2

Compound (I-c) (Compound (I) in which R³ is lower alkyl or allyl] can be prepared by the following reaction step.

Compound (I-c) is obtained from Compound (I-b) prepared by Process 1.

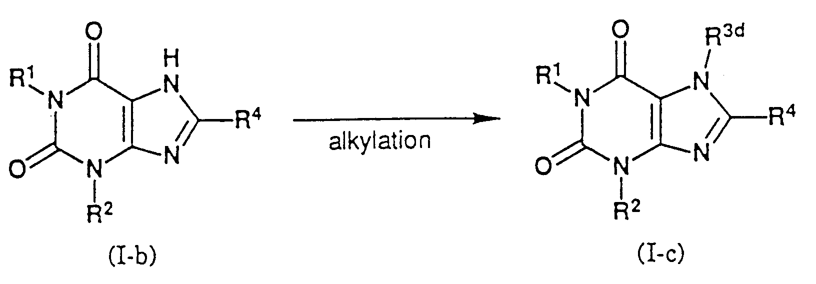

(In the formulae, R^{3d} represents lower alkyl or allyl in the definition of R³; and R¹, R², and R⁴ have the same meanings as defined above.)

Compound (I-c) can be obtained by reaction of Compound (I-b) with an alkylating agent, in the presence of a base if necessary. Examples of the alkylating agent are alkyl halides such as methyl iodide and allyl bromide, dialkyl sulfates such as dimethyl sulfate, sulfonic esters such as allyl p-toluenesulfonate, and diazoalkanes such as diazomethane. Examples of the base are alkali metal carbonates such as sodium carbonate and potassium carbonate, alkali metal hydrides such as sodium hydride, and alkali metal alkoxides such as sodium methoxide and sodium ethoxide. The reaction is completed in 0.5 to 24 hours at 0 to 180°C.

### Process 3

Compound (I-e) (Compound (I) in which Z is phenyl having hydroxy as substituent(s)] can be alternatively prepared by the following reaction step.

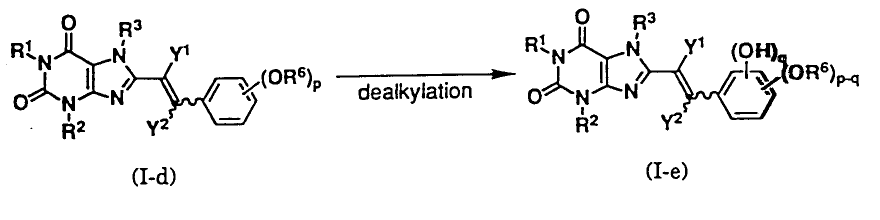

(In the formulae, R⁶ represents lower alkyl; p and q are integers of 1 to 3 and p ≧ q; and R¹, R², R³, Y¹, and Y² have the same meanings as defined above.)

The lower alkyl in the definition of R⁶ has the same meaning as defined above.

Compound (I-e) can be obtained by reaction of Compound (I-d) [Compound (I) in which Z is phenyl having lower alkoxy as substituent(s)] obtained by Process 1 or Process 2 with a dealkylating agent. Examples of the suitable dealkylating agent are boron tribromide and the complex of that with dimethyl disulfide, boron trichloride, iodotrimethylsilane, sodium ethanethiolate, sodium benzenethiolate, and hydrobromic acid. A reaction solvent selected from aromatic hydrocarbons such as toluene and xylene, halogenated hydrocarbons such as methylene chloride, chloroform, and dichloroethane, dimethylformamide, acetic acid, etc. depending upon the kind of the dealkylating agent is used. The reaction is completed in 10 minutes to 120 hours at -30 to 140°C.

### Process 4

Compound (I-f) [Compound (I) in which Z is phenyl having lower alkoxy as substituent(s)] can be alternatively prepared by the following reaction step.

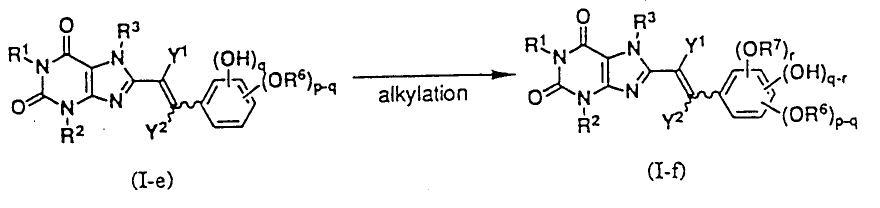

(In the formulae, R⁷ represents lower alkyl; r is an integer of 1 to 3 and q ≥ r; and R¹, R², R³, R⁶, Y¹, Y², p, and q have the same meanings as defined above.)

The lower alkyl in the definition of R⁷ has the same meaning as defined above.

Compound (I-f) can be obtained from Compound (I-e) according to the method of Process 2.

The desired compounds in the processes described above can be isolated and purified by purification methods conventionally used in synthetic organic chemistry, for example, filtration, extraction, washing, drying, concentration, recrystallization, and various kinds of chromatography.

In the case where a salt of Compound (I) is desired and it is produced in the form of the desired salt, it can be subjected to purification as such. In the case where Compound (I) is produced in the free state and its salt is desired, Compound (I) is dissolved or suspended in a suitable solvent, followed by addition of an acid or a base to form a salt.

Compounds (I) and pharmaceutically acceptable salts thereof may be in the form of adducts with water or various solvents, which can also be used as the therapeutic agent of the present invention.

Examples of Compounds (I) are shown in Table 1, and the structures thereof are shown in Table 2.

**Table 1-1**

| Compound No. | Name of the Compound |
|---|---|
| 1 | (E)-8-(3,4-dimethoxystyryl)-7-methyl-1,3-dipropylxanthine |
| 2 | (E)-8-(3,4,5-trimethoxystyryl)caffeine |
| 3 | (E) -7-methyl-1,3-dipropyl-8-styrylxanthine |
| 4 | (E)-1,3-diethyl-7-methyl-8-(3,4,5-trimethoxystyryl)xanthine |
| 5 | (E)-7-methyl-1,3-dipropyl-8-(3,4,5-trimethoxystyryl)xanthine |
| 6 | (E)-8-(4-methoxystyryl)-7-methyl-1,3-dipropylxanthine |
| 7 | (E)-1,3-diallyl-7-methyl-8-(3,4,5-trimethoxystyryl)xanthine |
| 8 | (E)-1,3-dibutyl-7-methyl-8-(3,4,5-trimethoxystyryl)xanthine |
| 9 | (E)-1,3-dipropyl-8-(3,4,5-trimethoxystyryl) xanthine |
| 10 | (E)-8-(3,4,5-trimethoxystyryl)theophylline |
| 11 | (E)-1,3-diallyl-8-(3,4,5-trimethoxystyryl) xanthine |
| 12 | (E)-8-(4-methoxy-2,3-dimethylstyryl)-1,3-dipropylxanthine |
| 13 | (E)-8-(4-methoxy-2,3-dimethylstyryl)-7-methyl-1,3-dipropylxanthine |
| 14 | (E)-8-(2,4-dimethoxy-3-methylstyryl)-1,3-dipropylxanthine |
| 15 | (E)-8-(2,4-dimethoxy-3-methylstyryl)-7-methyl-1,3-dipropylxanthine |
| 16 | (E)-8-[2-(1,4-benzodioxan-6-yl)vinyl]-1,3-dipropylxanthine |

**Table 1-2**

| Compound No. | Name of the Compound |
|---|---|
| 17 | (E)-8-[2-(1,4-benzodioxan-6-yl)vinyl]-7-methyl-1,3-dipropylxanthine |
| 18 | (E)-8-(3,4-methylenedioxystyryl)-1,3-dipropylxanthine |
| 19 | (E)-7-methyl-8-(3,4-methylenedioxystyryl)-1,3-dipropylxanthine |
| 20 | (E)-1,3-dipropyl-8-(2,3,4-trimethoxystyryl)xanthine |
| 21 | (E)-7-methyl-1,3-dipropyl-8-(2,3,4-trimethoxystyryl)xanthine |
| 22 | (E)-1,3-dipropyl-8-(2,4,5-trimethoxystyryl)-xanthine |
| 23 | (E)-7-methyl-1,3-dipropyl-8-(2,4,5-trimethoxystyryl)xanthine |
| 24 | (E)-8-(2,4-dimethoxystyryl)-1,3-dipropylxanthine |
| 25 | (E)-8-(2,4-dimethoxystyryl)-7-methyl-1,3-dipropylxanthine |
| 26 | (E)-8-(4-benzyloxy-3,5-dimethoxystyryl)-1,3-dipropylxanthine |
| 27 | (E)-8-(4-benzyloxy-3,5-dimethoxystyryl)-7-methyl-1,3-dipropylxanthine |
| 28 | (E)-8-(2,3-dimethoxystyryl)-1,3-dipropylxanthine |
| 29 | (E)-8-(2,3-dimethoxystyryl)-7-methyl-1,3-dipropylxanthine |
| 30 | (E)-8-(3,4-dimethylstyryl)-1,3-dipropylxanthine |
| 31 | (E)-8-(3,4-dimethylstyryl)-7-methyl-1,3-dipropylxanthine |
| 32 | (E)-8-(3,5-dimethoxystyryl)-1,3-dipropylxanthine |

**Table 1-3**

| Compound No. | Name of the Compound |
|---|---|
| 33 | (E)-8-(3,5-dimethoxystyryl)-7-methyl-1,3-dipropylxanthine |
| 34 | (E)-8-(3-nitrostyryl)-1,3-dipropylxanthine |
| 35 | (E)-7-methyl-8-(3-nitrostyryl)-1,3-dipropylxanthine |
| 36 | (E)-8-(3-fluorostyryl)-1,3-dipropylxanthine |
| 37 | (E)-8-(3-fluorostyryl)-7-methyl-1,3-dipropylxanthine |
| 38 | (E)-8-(3-chlorostyryl)-1,3-dipropylxanthine |
| 39 | (E)-8-(3-chlorostyryl)-7-methyl-1,3-dipropylxanthine |
| 40 | (E)-8-(2-chlorostyryl)-1,3-dipropylxanthine |
| 41 | (E)-8-(2-chlorostyryl)-7-methyl-1,3-dipropylxanthine |
| 42 | (E)-8-(2-fluorostyryl)-1,3-dipropylxanthine |
| 43 | (E)-8-(2-fluorostyryl)-7-methyl-1,3-dipropylxanthine |
| 44 | (E)-8-(4-methoxy-2,5-dimethylstyryl)-1,3-dipropylxanthine |
| 45 | (E)-8-(4-methoxy-2,5-dimethylstyryl)-7-methyl-1,3-dipropylxanthine |
| 46 | (Z)-8-(3,4-dimethoxystyryl)-7-methyl-1,3-dipropylxanthine |
| 47 | (E)-8-(4-ethoxystyryl)-1,3-dipropylxanthine |
| 48 | (E)-8-(4-ethoxystyryl)-7-methyl-1,3-dipropylxanthine |

**Table 1-4**

| Compound No. | Name of the Compound |
|---|---|
| 49 | (E)-8-(4-propoxystyryl)-1,3-dipropylxanthine |
| 50 | (E)-7-methyl-8-(4-propoxystyryl)-1,3-dipropylxanthine |
| 51 | (E)-8-(4-butoxystyryl)-1,3-dipropylxanthine |
| 52 | (E)-8-(4-butoxystyryl)-7-methyl-1,3-dipropylxanthine |
| 53 | (E)-8-(3,4-dihydroxystyryl)-7-methyl-1,3-dipropylxanthine |
| 54 | (E)-8-(3,4-diethoxystyryl)-7-methyl-1,3-dipropylxanthine |
| 55 | (E)-8-(3-bromo-4-methoxystyryl)-1,3-dipropylxanthine |
| 56 | (E)-8-(3-bromo-4-methoxystyryl)-7-methyl-1,3-dipropylxanthine |
| 57 | (E)-8-(2-bromo-4,5-dimethoxystyryl)-1,3-dipropylxanthine |
| 58 | (E)-8-(2-bromo-4,5-dimethoxystyryl)-7-methyl-1,3-dipropylxanthine |
| 59 | (E)-8-(3-bromo-4,5-dimethoxystyryl)-1,3-dipropylxanthine |
| 60 | (E)-8-(3-bromo-4,5-dimethoxystyryl)-7-methyl-1,3-dipropylxanthine |
| 61 | (E)-8-[2-(4-methoxynaphthyl)vinyl]-1,3-dipropylxanthine |
| 62 | (E)-8-[2-(4-methoxynaphthyl)vinyl]-7-methyl-1,3-dipropylxanthine |
| 63 | (E)-8-(3-hydroxy-4-methoxystyryl)-7-methyl-1,3-dipropylxanthine |

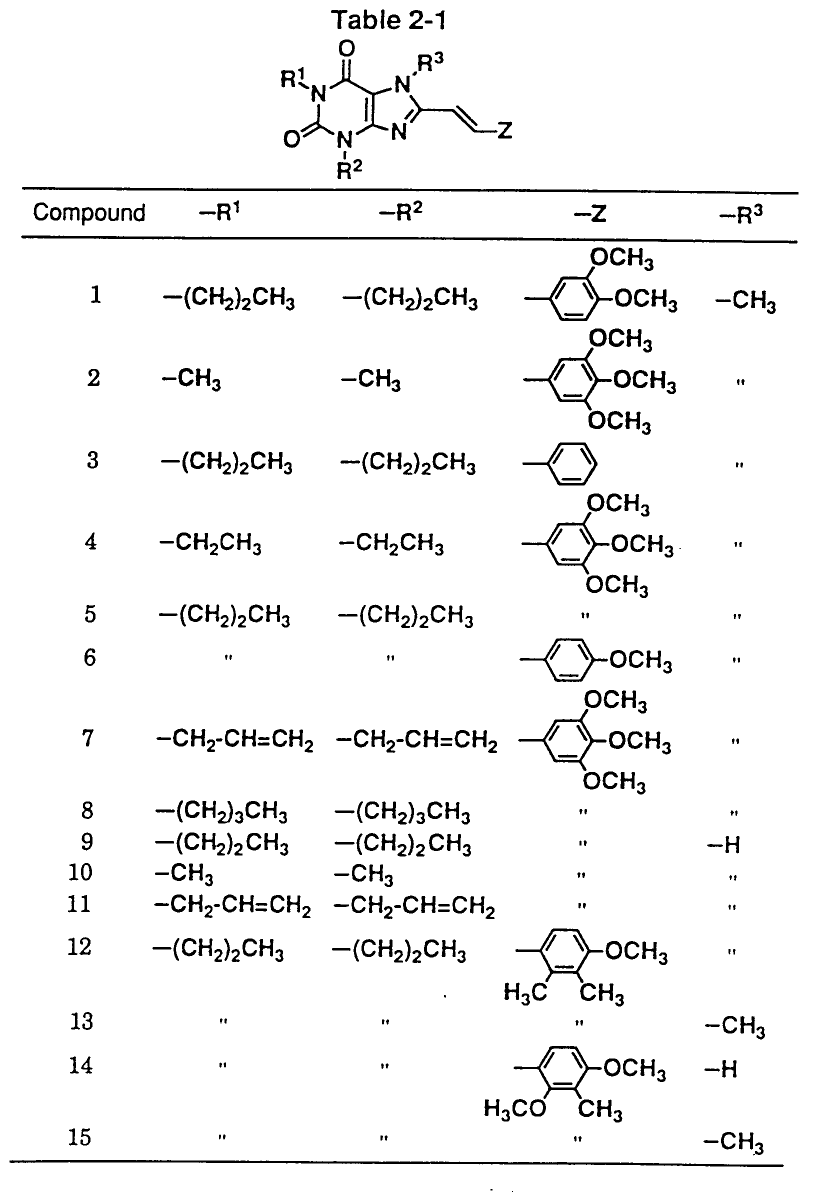

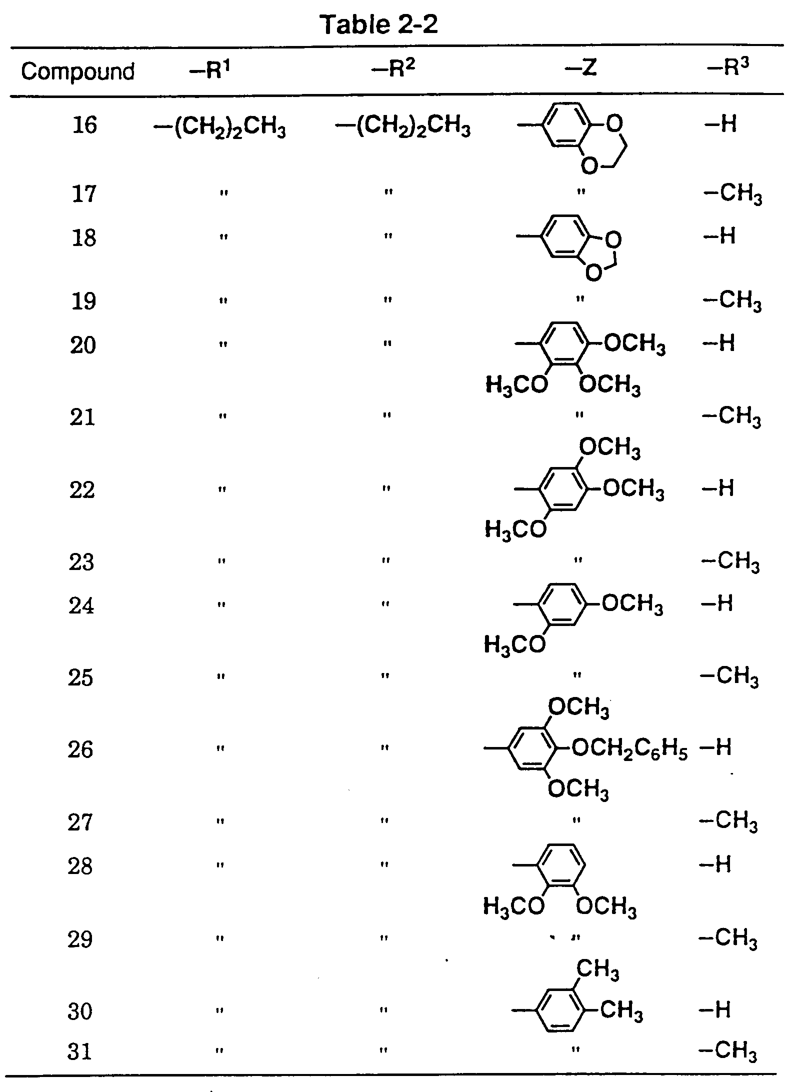

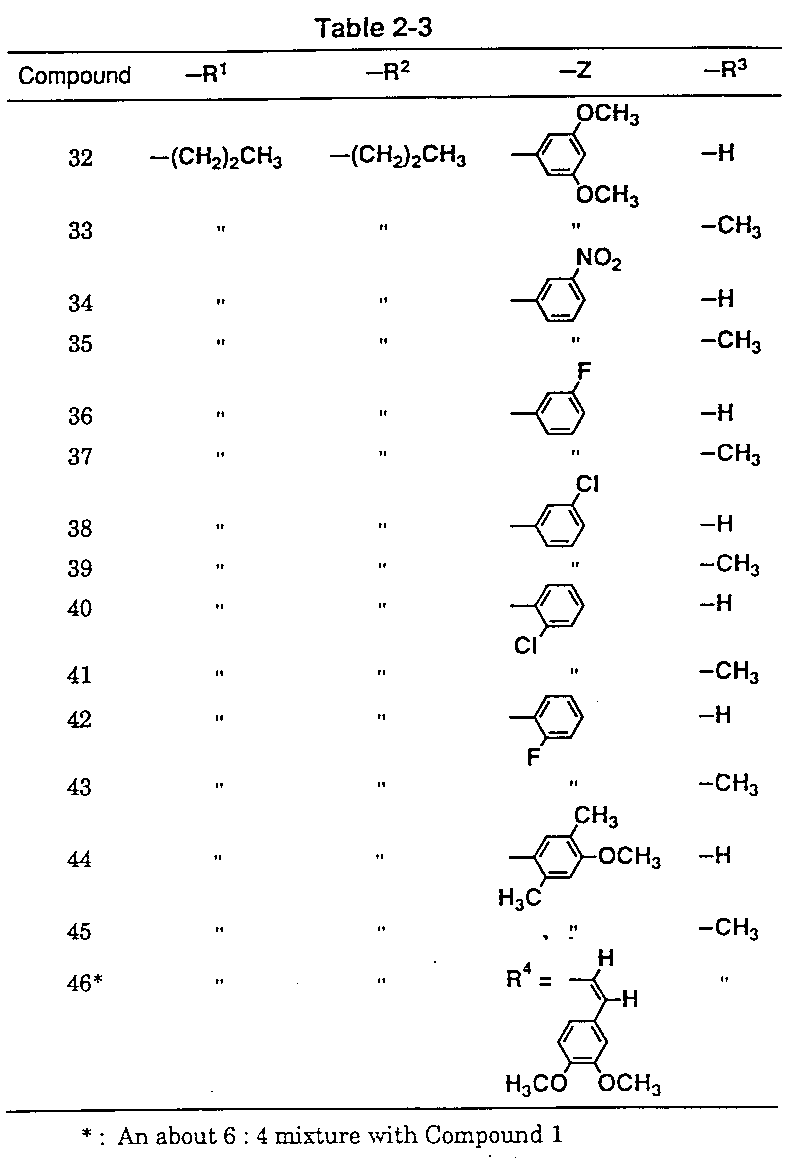

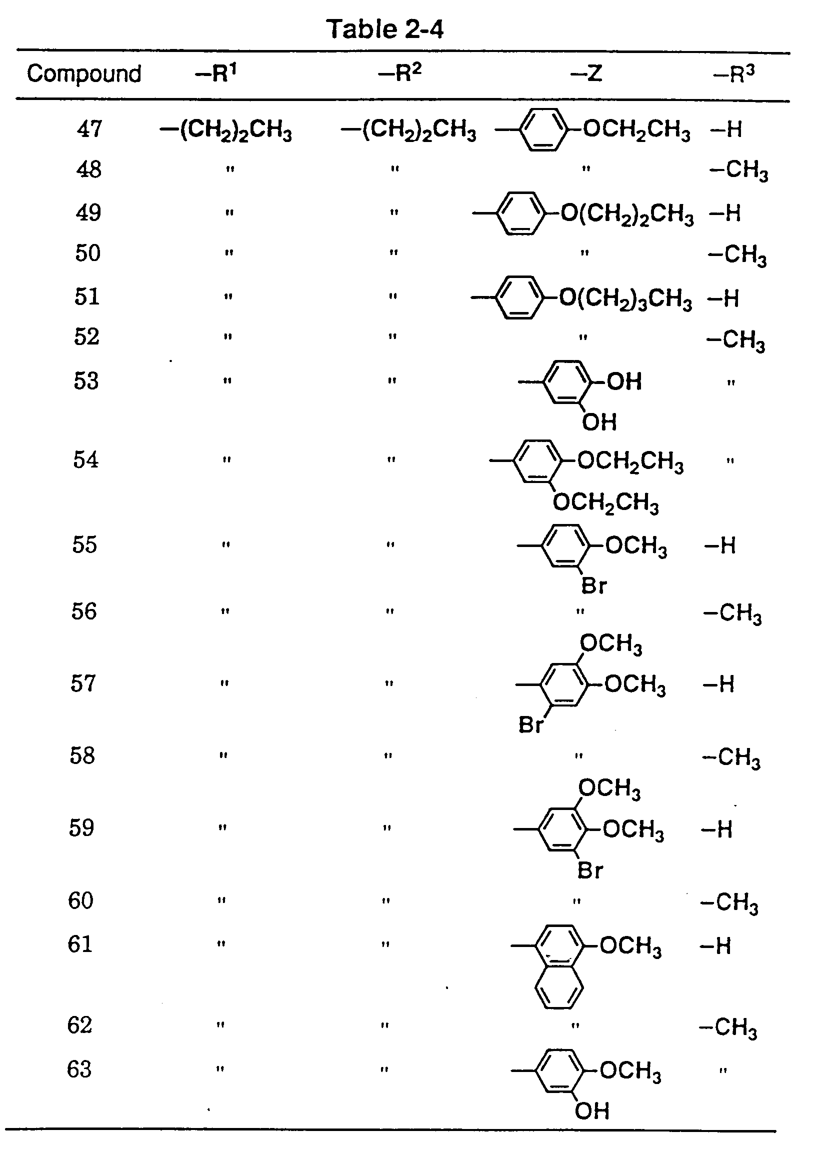

The pharmacological activities of Compounds (I) are shown below by experimental examples.

### Experimental Example 1 Effect on Locomotor Activity of Parkinson's Disease Model in Mouse

1-Methyl-4-phenyl-1,2,3,6-tetrahydropyridine (MPTP) causes symptoms of Parkinson's disease in humans [Science, 219, 979 (1983)]. It is reported that an experimental Parkinson's disease model was obtained by administering MPTP to mice [Science, 224, 1451 (1984)]. If a compound is effective on the experimental Parkinson's disease model in mouse, the compound can be expected to have a therapeutic effect on Parkinson's disease.

The experiment was performed by using several groups of 7-weeks-old male C57BL/6 mice (weighing 20 to 21 g, Japan SLC), each group consisting of 8 mice. MPTP (Aldrich Chemical Co., Inc.) dissolved in a physiological saline solution (Otsuka Pharmaceutical Co., Ltd.) was intraperitoneally administered to each mouse once a day for five consecutive days at a dose of 30 mg/kg. A test compound was suspended in injectable distilled water (Otsuka Phamaceutical Co., Ltd.) containing Tween 80 [polyoxyethylene (20) sorbitan monooleate]. L-DOPA (Kyowa Hakko Kogyo Co., Ltd.) was suspended in 0.3% CMC (sodium carboxylmethylcellulose). Thirty minutes after the final MPTP administration, the test compound suspensions and the control suspension [injectable distilled water (Otsuka Pharmaceutical Co., Ltd.) containing Tween 80] containing no test compound were orally administered to separate groups of the mice (0.1 ml per 10 g of body weight). The amount of active movements (horizontal activity) of each mouse was measured by using Automex-II (Columbus Instruments International Corp.) for the period of 30 minutes starting 30 minutes after the administration of the test compound. The effect of the compounds was evaluated by comparing the average counts of the active movements of the test compound-administered groups with those of the control groups. A significant difference test was performed by using Student's t-test.

The results are shown in Tables 3-1 to 3-5.

**Table 3-1**

| Group | Administration | | Dose of Test Compound (mg/kg) | Amount of Active Movements (average count ± S.E.M) |
|---|---|---|---|---|
| Normal Control | MPTP | (-) | | |
| | Test Compound | (-) | - | 1875 ± 77.7 |
| MPTP | MPTP | (+) | | |
| | Test Compound | (-) | - | 207 ± 85.5 |
| Compound 1 | MPTP | (+) | | |
| | Compound 1 | (+) | 10 | 628 ±174.5 * |
| Compound 2 | MPTP | (+) | | |
| | Compound 2 | (+) | 10 | 1134 ±267.0 * |
| L-DOPA | MPTP | (+) | | |
| | L-DOPA | (+) | 300 | 561 ±271.0 |

| | | | | |
|---|---|---|---|---|
| * p<0.05 | | | | |

**Table 3-2**

| Group | Administration | | Dose of Test Compound (mg/kg) | Amount of Active Movements (average count ± S.E.M) |
|---|---|---|---|---|
| Normal Control | MPTP | (-) | | |
| | Test Compound | (-) | - | 2185 ±156.2 |
| MPTP | MPTP | (+) | | |
| | Test Compound | (-) | - | 38 ± 24.2 |
| Compound 3 | MPTP | (+) | | |
| | Compound 3 | (+) | 40 | 228 ± 82.6 |
| Compound 4 | MPTP | (+) | | |
| | Compound 4 | (+) | 10 | 961 ±164.7 * |

| | | | | |
|---|---|---|---|---|
| * p<0.05 | | | | |

**Table 3-3**

| Group | Administration | | Dose of Test Compound (mg/kg) | Amount of Active Movements (average count ± S.E.M) |
|---|---|---|---|---|
| Normal Control | MPTP | (-) | | |
| | Test Compound | (-) | - | 2255 ±203.1 |
| MPTP | MPTP | (+) | | |
| | Test Compound | (-) | - | 17 ± 4.9 |
| Compound 5 | MPTP | (+) | | |
| | Compound 5 | (+) | 10 | 24 ± 6.5 |
| Compound 6 | MPTP | (+) | | |
| | Compound 6 | (+) | 10 | 34 ± 12.1 |
| Compound 7 | MPTP | (+) | | |
| | Compound 7 | (+) | 10 | 78 ± 48.3 |

**Table 3-4**

| Group | Administration | | Dose of Test Compound (mg/kg) | Amount of Active Movements (average count ± S.E.M) |
|---|---|---|---|---|
| Normal Control | MPTP | (-) | | |
| | Test Compound | (-) | - | 2032 ±167.4 |
| MPTP | MPTP | (+) | | |
| | Test Compound | (-) | - | 55 ± 16.8 |
| Compound 5 | MPTP | (+) | | |
| | Compound 5 | (+) | 40 | 217 ± 84.2 |
| Compound 6 | MPTP | (+) | | |
| | Compound 6 | (+) | 40 | 458 ±153.7 * |
| Compound 7 | MPTP | (+) | | |
| | Compound 7 | (+) | 40 | 310 ±119.5 |

| | | | | |
|---|---|---|---|---|
| * p<0.05 | | | | |

**Table 3-5**

| Group | Administration | | Dose of Test Compound (mg/kg) | Amount of Active Movements (average count ± S.E.M) |
|---|---|---|---|---|
| Normal Control | MPTP | (-) | | |
| | Test Compound | (-) | - | 2252 ±210.1 |
| MPTP | MPTP | (+) | | |
| | Test Compound | (-) | - | 18 ± 8.4 |
| Compound 9 | MPTP | (+) | | |
| | Compound 9 | (+) | 40 | 41 ± 18.0 |
| Compound 10 | MPTP | (+) | | |
| | Compound 10 | (+) | 40 | 32 ± 21.2 |
| Compound 11 | MPTP | (+) | | |
| | Compound 11 | (+) | 40 | 20 ± 7.1 |
| Compound 8 | MPTP | (+) | | |
| | Compound 8 | (+) | 40 | 43 ± 28.3 |

### Experimental Example 2 Effect on Haloperidol-Induced Catalepsy

The experiment was performed by using several groups of 5-weeks-old male ddY mice (weighing 22 to 24 g, Japan SLC), each group consisting of 5 mice. Haloperidol (Janssen Pharmaceutica) suspended in 0.3% CMC was intraperitoneally administered to each mouse at a dose of 1.0 mg/kg. Test compounds were suspended in 0.3% CMC or in injectable distilled water (Otsuka Pharmaceutical Co., Ltd.) containing Tween 80. L-DOPA (Kyowa Hakko Kogyo Co., Ltd.) and benserazide hydrochloride (Kyowa Hakko Kogyo Co., Ltd.) were suspended in 0.3% CMC. One hour after the haloperidol administration, the test compound suspensions and the control suspension [0.3% CMC or injectable distilled water (Otsuka Pharmaceutical Co., Ltd.) containing Tween 80] containing no test compound were orally administered to separate groups of the mice (0.1 ml per 10 g of body weight). One hour after the administration of the test compound, the forelimbs of each mouse and subsequently the hindlimbs of the same mouse were placed on a 4.5 cm-high, 1.0 cm-wide bar and catalepsy was estimated. All of the test compounds were orally administered at a dose of 10 mg/kg, and L-DOPA (100 mg/kg) and benserazide (25 mg/kg) were intraperitoneally administered together as a control experiment. The catalepsy score and the standard of judgment are shown below.

| score | duration of the cataleptic posture | |
|---|---|---|
| 0: | forelimbs | less than 5 seconds |
| | hindlimbs | less than 5 seconds |
| 1: | forelimbs | from 5 (inclusive) to 10 (exclusive) seconds |
| | hindlimbs | less than 5 seconds |
| 2: | forelimbs | 10 seconds or more |
| | hindlimbs | less than 5 seconds |
| 3: | forelimbs | from 5 (inclusive) to 10 (exclusive) seconds |
| | hindlimbs | from 5 (inclusive) to 10 (exclusive) seconds; |
| | or forelimbs | less than 5 seconds |
| | hindlimbs | 10 seconds or more |
| 4: | forelimbs | 10 seconds or more |
| | hindlimbs | from 5 (inclusive) to 10 (exclusive) seconds; |
| | or forelimbs | from 5 (inclusive) to 10 (exclusive) seconds |
| | hindlimbs | 10 seconds or more |
| 5: | forelimbs | 10 seconds or more |
| | hindlimbs | 10 seconds or more |

The effect of the compounds was evaluated by the total of the catalepsy scores of five mice in each group (25 points at the full). The groups wherein the total score was not more than 20 points were estimated to be effective. The number of the animals showing remission against catalepsy is the number of the mice for which the catalepsy score was not more than 4 points. The remission rate shows the rate of decrease in total score based on that of the control group.

The results are shown in Table 4.

**Table 4-1**

| Compound | Total Score | Number of the Animals Showing Remission | Remission Rate (%) |
|---|---|---|---|
| 0.3% CMC (Control) | 25 | 0 | |
| L-DOPA + benserazide | 20 | 3 | 20 |
| 1 | 13 | 5 | 48 |
| 2 | 11 | 5 | 56 |
| 3 | 20 | 4 | 20 |
| 4 | 20 | 4 | 20 |
| 5 | 18 | 4 | 28 |
| 6 | 19 | 3 | 24 |
| 7 | 13 | 4 | 48 |
| 11 | 20 | 3 | 20 |
| L-DOPA + benserazide | 18 | 4 | 28 |
| 13 | 5 | 5 | 80 |
| 15 | 19 | 4 | 24 |
| 16 | 20 | 4 | 20 |
| 18 | 20 | 4 | 20 |
| 19 | 19 | 3 | 24 |
| 20 | 19 | 3 | 24 |
| 23 | 18 | 4 | 28 |
| 24 | 19 | 4 | 24 |

**Table 4-2**

| Compound | Total Score | Number of the Animals Showing Remission | Remission Rate (%) |
|---|---|---|---|
| 0.3% Tween 80 (Control) | 25 | 0 | |
| L-DOPA + benserazide | 18 | 4 | 28 |
| 25 | 12 | 5 | 52 |
| 31 | 18 | 4 | 28 |
| 48 | 6 | 5 | 76 |
| 50 | 19 | 3 | 24 |
| 53 | 20 | 4 | 20 |
| 59 | 19 | 5 | 24 |

### Experimental Example 3 Acute Toxicity Test

Test compounds were orally administered to groups of dd-strain male mice weighing 20±1 g, each group consisting of three mice. Seven days after the administration, minimum lethal dose (MLD) of each compound was determined by observing the mortality.

The results are shown in Table 5.

**Table 5**

| Compound | MLD (mg/kg) | Compound | MLD (mg/kg) |
|---|---|---|---|
| 1 | > 300 | 33 | > 100 |
| 2 | > 300 | 34 | > 100 |
| 3 | > 300 | 35 | > 100 |
| 4 | > 300 | 36 | > 100 |
| 5 | > 300 | 37 | > 100 |
| 6 | > 300 | 38 | > 100 |
| 7 | > 300 | 39 | > 100 |
| 8 | > 300 | 40 | > 100 |
| 9 | > 300 | 41 | > 100 |
| 10 | > 300 | 42 | > 100 |
| 11 | > 300 | 43 | > 100 |
| 12 | > 300 | 44 | > 300 |
| 13 | > 300 | 45 | > 300 |
| 14 | > 100 | 46 | > 300 |
| 15 | > 300 | 47 | > 100 |
| 16 | > 300 | 48 | > 100 |
| 17 | > 300 | 49 | > 100 |
| 18 | > 300 | 50 | > 100 |
| 19 | > 300 | 51 | > 100 |
| 20 | > 300 | 52 | > 100 |
| 21 | > 300 | 53 | > 100 |
| 22 | > 300 | 54 | > 100 |
| 23 | > 300 | 55 | > 100 |
| 24 | > 100 | 56 | > 100 |
| 25 | > 300 | 57 | > 300 |
| 26 | > 100 | 58 | > 300 |
| 27 | > 100 | 59 | > 300 |
| 28 | > 100 | 60 | > 100 |
| 29 | > 300 | 61 | > 100 |
| 30 | > 100 | 62 | > 100 |
| 31 | > 100 | 63 | > 100 |
| 32 | > 100 | | |

As shown in Table 5, the MLD value of all the compounds are greater than 300 mg/kg, indicating that the toxicity of the compounds is weak. Therefore, these compounds can be safely used in a wide range of doses.

As described above, Compounds (I) and pharmaceutically acceptable salts thereof exhibit anti-Parkinson's syndrome effects. Thus, they are effective as therapeutic agents for Parkinson's disease. Compounds (I) and pharmaceutically acceptable salts thereof can be administered as they are, or in the form of various pharmaceutical compositions. The pharmaceutical compositions in accordance with the present invention can be prepared by uniformly mixing an effective amount of Compound (I) or a pharmaceutically acceptable salt thereof, as an active ingredient, with a pharmaceutically acceptable carrier. It is desired that such pharmaceutical compositions are prepared in a unit dose form suitable for oral administration or administration through injection.

For preparing a pharmaceutical composition for oral administration, any useful pharmaceutically acceptable carrier can be used. For example, liquid preparations for oral administration such as suspension and syrup can be prepared using water, sugars such as sucrose, sorbitol and fructose, glycols such as polyethylene glycol and propylene glycol, oils such as sesame oil, olive oil and soybean oil, preservatives such as p-hydroxybenzoates, flavors such as strawberry flavor and peppermint, and the like. Powders, pills, capsules and tablets can be prepared using excipients such as lactose, glucose, sucrose and mannitol, disintegrating agents such as starch and sodium alginate, lubricants such as magnesium stearate and talc, binders such as polyvinyl alcohol, hydroxypropyl cellulose and gelatin, surfactants such as fatty acid esters, plasticizers such as glycerin, and the like. Tablets and capsules are most useful oral unit dose forms because of the readiness of administration. For preparing tablets and capsules, solid pharmaceutical carriers are used.

Injectable preparations can be prepared using a carrier such as distilled water, a salt solution, a glucose solution or a mixture of a salt solution and a glucose solution. The preparations can be prepared in the form of solution, suspension or dispersion according to a conventional method by using a suitable auxiliary.

Compounds (I) and pharmaceutically acceptable salts thereof can be administered orally in the said dosage forms or parenterally as injections. The effective dose and the administration schedule vary depending upon mode of administration, age, body weight and conditions of a patient, etc. However, generally, Compound (I) or a pharmaceutically acceptable salt thereof is administered in a daily dose of 0.01 to 25 mg/kg in 3 to 4 parts.

Certain embodiments of the invention are illustrated in the following Examples, including the separately numbered series of "reference" Examples.

### Example 1

### (E)-8-(2-(1,4-Benzodioxan-6-yl)vinyl]-1,3-dipropyl-xanthine (Compound 16)

Substantially the same procedure as in Reference Example 1 was repeated using 1.35 g (5.96 mmol) of 5,6-diamino-1,3-dipropyluracil and 1.35 g (6.55 mmol) of 3-(1,4-benzodioxan-6-yl)acrylic acid. Then, the resultant crude crystals were recrystallised from ethanol/water to give 1.54 g (yield 65%) of Compound 16 as white needles.
Melting point: >275°C

| Elemental Analysis: C₂₁H₂₄N₄O₄ | | | |
|---|---|---|---|
| Calcd. (%): | C, 63.62; | H, 6.10; | N, 14.13 |
| Found (%): | C, 63.57; | H, 6.24; | N, 14.36 |

IR (KBr) ^{v}max (cm⁻¹); 1693, 1636, 1582, 1511
NMR (DMSO-D₆; 270 MHz) δ (ppm): 12.52(1H, brs), 7.63 (1H, d, J=16.2Hz), 7.10-7.06(2H, m), 6.95-6.86(2H, m), 4.29(4H, s), 4.15-4,10(4H m), 1.90-1.65(4H m), 1.05-0.95(6H, m)

### Example 2

### (E) -8- [2- (1,4-Benzodioxan-6-yl)vinyl]-7-methyl-1,3-dipropylxanthine (Compound 17)

Substantially the same procedure as in Reference Example 1 was repeated using 1.0 g (2.52 mmol) of Compound 16 obtained in Example 1 in place of Compound B. Then, the resultant crude crystals were recrystallized from ethanol to give 840 mg (yield 81%) of Compound 17 as pale yellow needles.
Melting Point: 181.9-182.3°C

| Elemental Analysis: C₂₂H₂₆N₄O₄ | | | |
|---|---|---|---|
| Calcd. (%): | C, 64.37; | H, 6.38; | N, 13.64 |
| Found (%): | C, 64.56; | H, 6.63; | N, 13.92 |

IR (KBr) νₘₐₓ (cm⁻¹) : 1693, 1651, 1510, 1288
NMR (CDCl₃; 270MHz) δ (ppm) : 7.67 (1H, d, J=15.5Hz), 7.10(2H, m), 6.88(1H, d, J=8.3Hz), 6.74(1H, d, J=15.5Hz), 4.30(4H, m), 4.13-3.95(4H, m), 4.03(3H, s), 1.88-1.65(4H, m), 1.03-0.94 (6H, m)

### Example 3

### (E)-8-(3,4-Methylenedioxystyryl)-1,3-dipropylxanthine (Compound 18)

Substantially the same procedure as in Reference Example 1 was repeated using 4.25 g (18.8 mmol) of 5,6-diamino-1,3-dipropyluracil and 4.33 g (22.6 mmol) of 3,4-methylenedioxycinnamic acid. Then, the resultant crude crystals were recrystallized from dioxane to give 4.92 g (yield 69%) of Compound 18 as a pale yellow powder.
Melting Point: >270°C

| Elemental Analysis: C₂₀H₂₂N₄O₄·0.75H₂O | | | |
|---|---|---|---|
| Calcd. (%): | C, 60.50; | H, 5.72; | N, 14.43 |
| Found (%): | C, 60.67; | H, 5.98; | N, 14.15 |

IR (KBr) νₘₐₓ (cm⁻¹) : 1688, 1648, 1499
NMR (DMSO-d₆; 270MHz) δ (ppm): 13.49(1H, brs), 7.56 (1H, d, J=16.3Hz), 7.30(1H, s), 7.07(1H, d, J=8.4Hz), 6.97-6.89(2H, m), 6.07(2H, s), 3.98 (2H, t, J=7.2Hz), 3.85(2H, t, J=7.3Hz), 1.75-1.35(4H, m), 0.95-0.80(6H, m)

### Example 4

### (E)-7-Methyl-8-(3,4-methylenedioxystyryl)-1,3-dipropylxanthine (Compound 19)

Substantially the same procedure as in Reference Example 1 was repeated using 3.0 g (7.85 mmol) of Compound 18 obtained in Example 3 in place of Compound B. Then, the resultant crude crystals were recrystallized from toluene/cyclohexane to give 2.33 g (yield 75%) of Compound 19 as a pale green powder.
Melting Point: 151.7-155.4°C

| Elemental Analysis: C₂₁H₂₄N₄O₄·0.25H₂O | | | |
|---|---|---|---|
| Calcd. (%): | C, 62.91; | H; 6.16; | N, 13.97 |
| Found (%): | C, 62.88; | H, 6.25; | N, 13.72 |

IR (KBr) νₘₐₓ (cm⁻¹): 1689, 1650, 1498, 1443
NMR (CDCl₃; 270MHz) δ (ppm) : 7.70 (1H, d, J=15.6Hz), 7.10-6.95(2H, m), 6.84 (1H, d, J=7.9Hz), 6.72(1H, d, J=15.6Hz), 6.02(2H, s), 4.10(2H, t, J=7.3Hz), 4.04(3H, s), 3.97(2H, t, J=7.3Hz), 1.90-1.65(4H, m), 1.05-0.90(6H, m)

### Example 5

### (E)-8-[2-(4-Methoxynaphthyl)vinyl]-1,3-dipropylxanthine (Compound 61)

Substantially the same procedure as in Reference Example 1 was repeated using 3.0 g (13.3 mmol) of 5,6-diamino-1,3-dipropyluracil and 3.33 g (14.6 mmol) of 3- (4-methoxynaphthyl)acrylic acid. Then, the resultant crude crystals were recrystallized from dioxane/water to give 3.12 g (yield 56%) of Compound 61 as yellow needles.
Melting Point: >280°C

| Elemental Analysis: C₂₄H₂₆N₄O₃ | | | |
|---|---|---|---|
| Calcd. (%): | C, 68.88; | H, 6.26; | N, 13.39 |
| Found (%): | C, 68.90; | H, 6.38; | N, 13.49 |

IR (KBr) νₘₐₓ (cm⁻¹): 1699, 1649, 1486, 1273
NMR (DMSO-d₆; 270MHz) δ (ppm): 13.58(1H, brs), 8.43 (1H, d, J=16.5Hz), 8.36(1H, d, J=8.6Hz), 8.24(1H, d, J=8.6Hz), 7.98(1H, d, J=7.8Hz), 7.70-7.54(2H, m), 7.12-7.06(2H, m), 4.03(3H, s), 4.02-3.86(4H, m), 1.79-1.56(4H, m), 0.92(3H, s), 0.89(3H, s)

### Example 6

### (E)-8-[2-(4-Methoxynaphthyl)vinyl]-7-methyl-1,3-dipropylxanthine (Compound 62)

Substantially the same procedure as in Reference Example 1 was repeated using 1.6 g (3.82 mmol) of Compound 61 obtained in Example 5 in place of Compound B. Then, the resultant crude crystals were recrystallized from ethyl acetate to give 1.25 g (yield 76%) of Compound 62 as pale yellow plates.
Melting Point: 212.6-213.9°C

| Elemental Analysis: C₂₅H₂₈N₄O₃ | | | |
|---|---|---|---|
| Calcd. (%): | C, 69.43; | H, 6.52; | N, 12.95 |
| Found (%): | C, 69.46; | H, 6.68; | N; 12.95 |

IR (KBr) νₘₐₓ (cm⁻¹): 1701, 1650, 1486, 1439, 1267
NMR (CDCl₃; 270MHz) δ (ppm): 8.52(1H, d, J=15.5Hz), 8.34(1H, d, J=8.3Hz), 8.23(1H, d, J=8.6Hz), 7.77 (1H, d, J=8.3Hz), 7.66-7.52(2H, m), 6.89(1H, d, J=15.5Hz), 6.87(1H, d, J=8.3Hz), 4.18-4.11(2H, m), 4.07(3H, s), 4.06(3H, s), 4.02-3.97(2H, m), 1.95-1.64(4H, m), 1.03(3H, t, J=7.3Hz), 0.98(3H, t, J=7.3Hz)

### Example 7 Tablets

Tablets having the following composition were prepared in a conventional manner.

Compound 1 (40 g) was mixed with 286.8 g of lactose and 60 g of potato starch, followed by addition of 120 g of a 10% aqueous solution of hydroxypropylcellulose. The resultant mixture was kneaded, granulated, and then dried by a conventional method. The granules were refined, thus obtaining granules used to make tablets. After mixing the granules with 1.2 g of magnesium stearate, the mixture was formed into tablets each containing 20 mg of the active ingredient by using a tablet maker (Model RT-15, Kikusui) having pestles of 8 mm diameter. The composition of each tablet thus prepared is shown in Table 6.

**Table 6**

| Composition of One Tablet | |
|---|---|
| Compound 1 | 20 mg |
| Lactose | 143.4 mg |
| Potato Starch | 30 mg |
| Hydroxypropylcellulose | 6 mg |
| Magnesium Stearate | 0.6 mg |
| | 200 mg |

### Example 8 Fine Granules

Fine granules having the following composition were prepared in a conventional manner.

Compound 2 (20 g) was mixed with 655 g of lactose and 285 g of corn starch, followed by addition of 400 g of a 10% aqueous solution of hydroxypropylcellulose. The resultant mixture was kneaded, granulated, and then dried by a conventional method, thus obtaining fine granules containing 20 g of the active ingredient in 1,000 g. The composition of one pack of the fine granules is shown in Table 7.

**Table 7**

| Composition of One Pack of Fine Granules | |
|---|---|
| Compound 2 | 20 mg |
| Lactose | 655 mg |
| Corn Starch | 285 mg |
| Hydroxypropylcellulose | 40 mg |
| | 1,000 mg |

### Example 9 Capsules

Capsules having the following composition were prepared in a conventional manner.

Compound 1 (200 g) was mixed with 995 g of Avicel and 5 g of magnesium stearate. The mixture was put in hard capsules No. 4 each having a capacity of 120 mg by using a capsule filler (Model LZ-64, Zanashi), thus obtaining capsules each containing 20 mg of the active ingredient. The composition of one capsule thus prepared is shown in Table 8.

**Table 8**

| Composition of One Capsule | |
|---|---|
| Compound 1 | 20 mg |
| Avicel | 99.5mg |
| Magnesium Stearate | 0.5mg |
| | 120 mg |

### Example 10 Injections

Injections having the following composition were prepared in a conventional manner.

Compound 2 (1 g) was dissolved in 100 g of purified soybean oil, followed by addition of 12 g of purified egg yolk lecithin and 25 g of glycerine for injection. The resultant mixture was made up to 1,000 ml with distilled water for injection, thoroughly mixed, and emulsified by a conventional method. The resultant dispersion was subjected to aseptic filtration by using 0.2 µm disposable membrane filters, and then aseptically put into glass vials in 2 ml portions, thus obtaining injections containing 2 mg of the active ingredient per vial. The composition of one injection vial is shown in Table 9.

**Table 9**

| Composition of One Injection Vial | |
|---|---|
| Compound 2 | 2 mg |
| Purified Soybean Oil | 200 mg |
| Purified Egg Yolk Lecithin | 24 mg |
| Glycerine for Injection | 50 mg |
| Distilled Water for Injection | 1.72 ml |
| | 2.00 ml |

### Reference Example 1

### (E)-8-(3,4-Dimethoxystyryl)-7-methyl-1,3-dipropylxanthine (Compound 1)

3,4-Dimethoxycinnamic acid (2.03 g, 9.74 mmol) and 3-(3-diethylaminopropyl)-1-ethylcarbodiimide hydrochloride (2.54 g, 13.3 mmol) were added to a mixture of water (60 ml) and dioxane (30 ml) containing 5,6-diamino-1,3-dipropyluracil (U.S. Patent No. 2,602,795) (2.00 g, 8.85 mmol). The resultant solution was stirred at room temperature for 2 hours at pH 5.5. After neutralization, the reaction solution was extracted three times with 50 ml of chloroform. The combined extract was washed with a saturated aqueous solution of sodium chloride and dried over anhydrous sodium sulfate, followed by evaporation under reduced pressure. The residue was purified by silica gel column chromatography (eluent: 2% methanol/chloroform) to give 3.47 g (yield 94%) of (E)-6-amino-5-(3,4-dimethoxycinnamoyl)amino-1,3-dipropyluracil (Compound A) as an amorphous substance.
- NMR (CDCl₃; 90MHz) δ (ppm) :: 7.84(1H, brs), 7.50(1H, d, J=15.9Hz), 7.10-6.65(3H, m), 6.53(1H, d, J=15.9Hz), 5.75(2H, brs), 4.00-3.50(4H, m), 3.85(6H, brs), 2.00-1.40(4H, m), 1.10-0.80(6H, m)

To 3.38 g (8.13 mmol) of Compound A were added 40 ml of dioxane and 80 ml of an aqueous 1N sodium hydroxide solution, followed by heating under reflux for 10 minutes. After cooling, the solution was neutralized, and deposited crystals were collected by filtration. Then, the collected crystals were recrystallized from dimethylsulfoxide/water to give 2.49 g (yield 77%) of (E)-8-(3,4-dimethoxystyryl)-1,3-dipropylxanthine (Compound B) as white crystals.
Melting Point: 260.0-263.8°C

| Elemental Analysis: C₂₁H₂₆N₄O₄ | | | |
|---|---|---|---|
| Calcd. (%): | C, 63.30; | H, 6.57; | N, 14.06 |
| Found (%): | C, 63.29; | H, 6.79; | N, 14.21 |

IR (KBr) νₘₐₓ (cm⁻¹): 1701, 1640
NMR (DMSO-d₆; 270MHz) δ (ppm) : 13.39(1H, brs), 7.59 (1H, d, J=16.7Hz), 7.26(1H, d, J=1.8Hz), 7.13(1H, dd, J=1.8, 8.6Hz), 6.98(1H, d, J=8.6Hz), 6.95(1H, d, J=16.7Hz), 3. 99(2H, t), 4.00-3.85(2H, t), 3.83(3H, s), 3.80(3H, s), 1.80-1.55(4H, m), 1.00-0.85(6H, m)

Compound B (1.20 g, 3.02 mmol) was dissolved in 20 ml of dimethylformamide. To the solution were added 1.04 g (7.55 mmol) of potassium carbonate and subsequently 0.38 ml (6.04 mmol) of methyl iodide, and the resultant mixture was stirred at 50°C for 30 minutes. After cooling, insoluble matters were filtered off, and 400 ml of water was added to the filtrate. The mixture was extracted three times with 100 ml of chloroform. The extract was washed twice with water and once with a saturated aqueous solution of sodium chloride, and dried over anhydrous sodium sulfate, followed by evaporation under reduced pressure. The residue was purified by silica gel column chromatography (eluent: 1% methanol/chloroform), followed by recrystallization from propanol/water to give 1.22 g (yield 98%) of Compound 1 as white needles.
Melting Point: 164.1-166.3°C

| Elemental Analysis: C₂₂H₂₈N₄O₄ | | | |
|---|---|---|---|
| Calcd. (%): | C, 64.06; | H, 6.84; | N, 13.58 |
| Found (%): | C, 64.06; | H, 6.82; | N, 13.80 |

IR (KBr) νₘₐₓ (cm⁻¹): 1692, 1657
NMR (DMSO-d₆; 270MHz) δ (ppm): 7.60(1H, d, J=15.8Hz), 7.40(1H, d, 2.0Hz), 7.28(1H, dd, J=2.0, 8.4Hz), 7.18(1H, d, J=15.8Hz), 6.99(1H, d, J=8.4Hz), 4.02(3H, s), 3.99(2H, t), 3.90-3.80(2H, m), 3.85(3H, s), 3.80(3H, s), 1.80-1.55(4H, m), 1.00-0.85(6H, m)

### Reference Example 2

### (E)-7-Methyl-1,3-dipropyl-8-styrylxanthine (Compound 3)

5,6-Diamino-1,3-dipropyluracil (U.S. Patent No. 2,602,795) (6.0 g, 26.5 mmol) was slowly added to a mixture of methanol (360 ml) and acetic acid (15 ml) containing cinnamaldehyde (3.34 ml, 26.5 mmol) under ice cooling. The resultant mixture was stirred at room temperature for 30 minutes, followed by evaporation under reduced pressure to give 6.30 g (yield 70%) of (E)-6-amino-5-(3-phenyl-3-propenylidene)-1,3-dipropyluracil (Compound C) as an amorphous substance.
- Melting Point:: 159.5-161.0°C
- IR (KBr) νₘₐₓ (cm⁻¹):: 1687, 1593
- NMR (CDCl₃; 90MHz) δ (ppm) :: 9.75-9.60(1H, m), 7.60-7.25(5H, m), 7.00-6.80(2H, m), 5.70(brs, 2H), 4.00-3.70(4H, m), 2.00-1.40(4H, m), 1.10-0.75(6H, m)
- MS m/e (relative intensity):: 340(100, M⁺), 130(86)

To 6.30 g (18.5 mmol) of Compound C was added 240 ml of ethanol, and the mixture was heated under reflux for 2 hours in the presence of 4.32 g (26.5 mmol) of ferric chloride. After cooling, deposited crystals were collected by filtration to give 3.61 g (yield 61%) of (E)-1,3-dipropyl-8-styrylxanthine (Compound D) as white crystals.
Melting Point: 259.3-261.0°C (recrystallised from ethanol)

| Elemental Analysis: C₁₉H₂₂N₄O2 | | | |
|---|---|---|---|
| Calcd. (%): | C, 67.43; | H, 6.55; | N, 16.56 |
| Found (%): | C, 67.40; | H, 6.61; | N, 16.71 |

IR (KBr) νₘₐₓ (cm⁻¹): 1700, 1650, 1505
NMR (DMSO-d₆) δ (ppm): 13.59(1H, brs), 7.70-7.55 (3H, m), 7.50-7.30(3H, m), 7.06(1H, d, J= 16.5Hz), 3.99(2H, t), 3.86(2H, t), 2.80-2.50(4H, m), 0.95-0.80(6H, m)

Subsequently, the same procedure as in Reference Example 1 was repeated using Compound D in place of Compound B to give 1.75 g (yield 84%) of Compound 3 as white needles.
Melting Point: 162.8-163.2°C

| Elemental Analysis: C₂₀H₂₄N₄O₂ | | | |
|---|---|---|---|
| Calcd. (%): | C, 68.16; | H, 6.86; | N, 15.90 |
| Found (%): | C, 67.94; | H, 6.96; | N, 16.15 |

IR (KBr) νₘₐₓ (cm⁻¹): 1690, 1654, 1542, 1450, 1437
NMR (CDCl₃) δ (ppm): 7.79(1H, d, J=15.8Hz), 7.65-7.55(2H, m), 7.48-7.35(3H, m), 6.92(1H, d, J=15.8Hz), 4.11(2H, t), 4.06(3H, s), 3.98(2H, t), 2.00-1.60(4H, m), 1.08-0.95(6H, m)

### Reference Example 3

### (E)-1,3-Dipropyl-8-(3,4,5-trimethoxystyryl)xanthine (Compound 9)

3,4,5-Trimethoxycinnamic acid (5.78 g, 24.3 mmol) and 6.36 g (33.2 mmol) of 3-(3-diethylaminopropyl)-1-ethylcarbodiimide hydrochloride were added to a mixture of dioxane (150 ml) and water (75 ml) containing 5.00 g (22.1 mmol) of 5,6-diamino-1,3-dipropyluracil. The resultant solution was stirred at room temperature at pH 5.5 for one hour. After the reaction, the solution was adjusted to pH 7 and extracted three times with chloroform. The combined extract was washed with a saturated aqueous solution of sodium chloride and dried over anhydrous sodium sulfate, followed by evaporation under reduced pressure. The residue was purified by silica gel column chromatography (eluent: 3% methanol/chloroform) to give 8.06 g (yield 82%) of (E)-6-amino-1,3-dipropyl-5-(3,4,5-trimethoxycinnamoyl)aminouracil (Compound E) as an amorphous substance.
- NMR (CDCl₃; 90MHz) δ (ppm):: 7.85(1H, brs), 7.48(1H, d, J=15.6Hz), 6.67(2H, s), 6.56(1H, d, J=15.6Hz), 5.80(2H, brs), 4.00-3.70(4H, m), 3.89(9H, s), 1.80-1.45(4H, m), 1.15-0.80(6H, m)

To 10.02 g (22.5 mmol) of Compound E were added 100 ml of dioxane and 100 ml of an aqueous 2N sodium hydroxide solution, and the solution was heated under reflux for 10 minutes. After cooling, the solution was neutralized, and deposited crystals were collected by filtration. Then, the collected crystals were recrystallized from dioxane/water to give 6.83 g (yield 91%) of (E)-1,3-dipropyl-8-(3,4,5-trimethoxystyryl)xanthine (Compound 9) as white crystals.
Melting Point: 161.8-162.6°C

| Elemental Analysis: C₂₂H₂₈N₄O₅ | | | |
|---|---|---|---|
| Calcd. (%): | C, 61.66; | H, 6.58; | N, 13.07 |
| Found (%): | C, 61.73; | H, 6.37; | N, 13.08 |

IR (KBr) νₘₐₓ (cm⁻¹): 1702, 1643
NMR (CDCl₃; 90MHz) δ (ppm): 12.87(1H, brs), 7.72(1H, d, J=16.3Hz), 6.96(1H, d, J=16.3Hz), 6.81(2H, s), 4.30-3.95(4H, m), 3.92(6H, s), 3.90(3H, s), 2.10-1.50(4H, m), 1.02(2H, t), 0.90(2H, t)

### Reference Example 4

### (E)-7-Methyl-1,3-dipropyl-8-(3,4,5-trimethoxystyryl)xanthine (Compound 5)

The same procedure as in Reference Example 1 was repeated using Compound 9 in place of Compound B to give 1.75 g (yield 84%) of Compound 5 as white needles.
Melting Point: 168.4-169.1°C (recrystallized from ethanol/water)

| Elemental Analysis: C₂₃H₃₀N₄O₅ | | | |
|---|---|---|---|
| Calcd. (%): | C, 62.42; | H, 6.83; | N, 12.66 |
| Found (%): | C, 62.48; | H, 6.60; | N, 12.70 |

IR (KBr) νₘₐₓ (cm⁻¹): 1698, 1659
NMR (CDCl₃; 90MHz) δ (ppm): 7.71(1H, d, J=15.8Hz), 6.86(2H, s), 6.78(1H, d, J=15.8Hz), 4.30-3.95(4H, m), 4.07(3H, s), 3.93(6H, s), 3.90(3H, s), 2.05-1.50 (4H, m), 1.20-0.85 (6H, m)

### Reference Example 5

### (E)-8-(4-Methoxystyryl)-7-methyl-1,3-dipropylxanthine (Compound 6)

Substantially the same procedure as in Reference Example 1 was repeated using 2.00 g (8.85 mmol) of 5,6-diamino-1,3-dipropyluracil and 1.73 g (9.74 mmol) of 4-methoxycinnamic acid to give 2.29 g (overall yield 68%) of Compound 6.
Melting Point: 159.8-161.3°C (recrystallized from ethanol/water)

| Elemental Analysis: C₂₁H₂₆N₄O₃ | | | |
|---|---|---|---|
| Calcd. (%): | C, 65.94; | H, 6.85; | N, 14.64 |
| Found (%): | C, 65.92; | H, 6.90; | N, 14.88 |

IR (KBr) νₘₐₓ (cm⁻¹): 1695, 1658
NMR (DMSO-d₆) δ (ppm): 7.72 (2H, d, J=8. 8Hz), 7.61(1H, d, J=15.8Hz), 7.16(1H, d, J=15.8Hz), 4.05-3.95(2H, m), 4.00(3H, s), 3.83(2H, t), 3.80 (3H, s), 1.85-1.50 (4H, m), 1.00-0.85 (6H, m)

### Reference Example 6

### (E)-1,3-Diallyl-8-(3,4,5-trimethoxystyryl)xanthine (Compound 11)

Substantially the same procedure as in Reference Example 3 was repeated using 3.0 g (13.5 mmol) of 1,3-diallyl-5,6-diaminouracil and 3.55 g (14.9 mmol) of 3,4,5-trimethoxycinnamic acid to give 4.48 g (yield 75%) of (E)-1,3-diallyl-6-amino-5-(3,4,5-trimethoxycinnamoyl)aminouracil (Compound F) as an amorphous substance.
- NMR (CDCl₃; 90MHz) δ (ppm):: 7.90(1H, brs), 7.56(1H, d, J=16.0Hz), 6.71(2H, s), 6.57(1H, d, J=16.0Hz), 6.15-5.60(4H, m), 5.50-5.05(4H, m), 4.75-4.45(4H, m), 3.90(9H, s)

Substantially the same procedure as in Reference Example 3 was repeated using 4.34 g (9.82 mmol) of Compound F in place of Compound E to give 2.81 g (yield 68%) of Compound 11 as a pale yellowish green powder.
Melting Point: 253.1-255.4°C (recrystallized from dioxane)

| Elemental Analysis: C₂₂H₂₄N₄O₅·1/2H₂O | | | |
|---|---|---|---|
| Calcd. (%): | C, 60.96; | H, 5.81; | N, 12.93 |
| Found (%): | C, 61.05; | H, 5.60; | N, 12.91 |

IR (KBr) νₘₐₓ (cm⁻¹): 1704, 1645, 1583, 1510
NMR (CDCl₃) δ (ppm): 12.94(1H, brs), 7.73(1H, d, J=16.3Hz), 7.05(1H, d, J=16.3Hz), 6.81(2H, s), 6.12-5.92(2H, m), 5.37-5.22(4H, m), 4.83-4.76(4H, m), 3.91(6H, s), 3.90(3H, s)

### Reference Example 7

### (E)-1,3-Diallyl-7-methyl-8-(3,4,5-trimethoxystyryl)xanthine (Compound 7)

Substantially the same procedure as in Reference Example 1 was repeated using 1.13 g (2.67 mmol) of Compound 11 in place of Compound B to give 620 mg (yield 53%) of Compound 7 as pale yellow needles.
Melting Point: 189.0-191.1°C (recrystallized from ethyl acetate)

| Elemental Analysis: C₂₃H₂₆N₄O₅ | | | |
|---|---|---|---|
| Calcd. (%): | C, 63.00; | H, 5.97; | N, 12.77 |
| Found (%): | C, 63.00; | H, 6.05; | N, 12.85 |

IR (KBr) νₘₐₓ (cm⁻¹): 1699, 1660
NMR (CDCl₃; 90MHz) δ (ppm): 7.78(1H, d, J=16.0Hz), 6.85(2H, s), 6.84(1H, d, J=16.0Hz), 6.30-5.75(2H, m), 5.45-5.10(4H, m), 4.85-4.55(4H, m), 4.07(3H, s), 3.92(6H, s), 3.90(3H, s)

### Reference Example 8

### (E)-1,3-Dibutyl-7-methyl-8-(3,4,5-trimethoxystyryl)xanthine (Compound 8)

Substantially the same procedure as in Reference Example 1 was repeated using 4.75 g (18.7 mmol) of 5,6-diamino-1,3-dibutyluracil and 4.90 g (20.6 mmol) of 3,4,5-trimethoxycinnamic acid to give 5.49 g (overall yield 63%) of Compound 8 as a pale green powder.
Melting Point: 136.8-137.3°C (recrystallized from ethanol/water)

| Elemental Analysis: C₂₅H₃₄N₄O₅ | | | |
|---|---|---|---|
| Calcd. (%): | C, 63.81; | H, 7.28; | N, 11.91 |
| Found (%): | C, 63.63; | H, 6.93; | N, 11.99 |

IR (KBr) νₘₐₓ (cm⁻¹): 1692, 1659
NMR (CDCl₃; 90MHz) δ (ppm): 7.68(1H, d, J=15.8Hz), 6.80(2H, s), 6.79(1H, d, J=15.8Hz), 4.30-3.90(4H, m), 4.03(3H, s), 3.95(6H, s), 3.91(3H, s), 1.90-1.10 (8H, m), 1.05-0.80 (6H, m)

### Reference Example 9

### (E)-8-(4-Methoxy-2,3-dimethylstyryl)-1,3-dipropylxanthine (Compound 12)

Substantially the same procedure as in Reference Example 1 was repeated using 2.31 g (10.24 mmol) of 5,6-diamino-1,3-dipropyluracil and 2.42 g (15.4 mmol) of 4-methoxy-2,3-dimethylcinnamic acid. Then, the resultant crude crystals were recrystallized from dioxane/water to give 1.96 g (yield 48%) of Compound 12 as a white powder.
Melting Point: 270.7-271.3°C

| Elemental Analysis: C₂₂H₂₈N₄O₃ | | | |
|---|---|---|---|
| Calcd. (%): | C, 66.64; | H, 7.11; | N, 14.13 |
| Found (%): | C, 66.68; | H, 7.20; | N, 14.04 |

IR (KBr) νₘₐₓ (cm⁻¹): 1704, 1650, 1591, 1269
NMR (DMSO-d₆; 270MHz) δ (ppm): 7.93(1H, d, J=16.3Hz), 7.57(1H, d, J=8.9Hz), 6.88(1H, d, J=8.9Hz), 6.82(1H, d, J=16.3Hz), 3.98(2H, t, J=7.1Hz), 3.86(2H, t, J=7.3Hz), 3.81(3H, s), 2.32(3H, s), 2.09(3H, s), 1.80-1.55(4H, m), 0.95-0.80(6H, m)

### Reference Example 10

### (E)-8-(4-Methoxy-2,3-dimethylstyryl)-7-methyl-1,3-dipropylxanthine (Compound 13)

Substantially the same procedure as in Reference Example 1 was repeated using 4.00 g (5.10 mmol) of Compound 12 obtained in Reference Example 9 in place of Compound B to give 1.73 g (yield 83%) of Compound 13 as yellow needles.
Melting Point: 171.0-173.5°C

| Elemental Analysis: C₂₃H₃₀N₄O₃ | | | |
|---|---|---|---|
| Calcd. (%): | C, 67.29; | H, 7.36; | N, 13.64 |
| Found (%): | C, 66.87; | H, 7.67; | N, 13.51 |

IR (KBr) νₘₐₓ (cm⁻¹): 1697, 1659, 1593, 1493
NMR (CDCl₃; 270MHz) δ (ppm): 8.07(1H, d, J=15.3Hz), 7.46(1H, d, J=8.4Hz), 6.77(1H, d, J=8.4Hz), 6.67(1H, d, J=15.3Hz), 4.12(2H, t, J=7.3Hz), 4.03(3H, s), 3.98(2H, t, J=7.3Hz), 3.86(3H, s), 2.39(3H, s), 2.26(3H, s), 1.85-1.50(4H, m), 1.05-0.90(6H, m)

### Reference Example 11

### (E)-8-(2,4-Dimethoxy-3-methylstyryl)-1,3-dipropylxanthine (Compound 14)

Substantially the same procedure as in Reference Example 1 was repeated using 1.25 g (5.52 mmol) of 5,6-diamino-1,3-dipropyluracil and 1.35 g (6.08 mmol) of 2,4-dimethoxy-3-methylcinnamic acid. Then, the resultant crude crystals were recrystallized from dioxane/water to give 1.14 g (yield 50%) of Compound 14 as white needles.
Melting Point: 255.2-256.0°C

| Elemental Analysis: C₂₂H₂₈N₄O₄ | | | |
|---|---|---|---|
| Calcd. (%): | C, 64.06; | H, 6.84; | N, 13.58 |
| Found (%): | C, 63.77; | H, 7.01; | N, 13.42 |

IR (KBr) νₘₐₓ (cm⁻¹): 1694, 1650, 1594, 1495
NMR (DMSO-d₆; 270MHz) δ (ppm): 13.54(1H, brs), 7.76 (1H, d, J=16.5Hz), 7.59(1H, d, J=8.9Hz), 6.99(1H, d, J=16.5Hz), 6.84(1H, d, J=8.9Hz), 3.99(2H, t, J=7.4Hz), 3.85(2H, t, J=7.3Hz), 3.83(3H, s), 3.70 (3H, s), 2.09(3H, s), 1.80-1.55(4H, m), 0.95-0.80 (6H, m)

### Reference Example 12

### (E)-8-(2,4-Dimethoxy-3-methylstyryl)-7-methyl-1,3-dipropylxanthine (Compound 15)

Substantially the same procedure as in Reference Example 1 was repeated using 1.10 g (2.67 mmol) of Compound 14 obtained in Reference Example 11 in place of Compound B. Then, the resultant crude crystals were recrystallized from ethanol/2-propanol to give 620 mg (yield 55%) of Compound 15 as pale yellow grains.
Melting Point: 191.4-191.8°C

| Elemental Analysis: C₂₃H₃₀N₄O₄ | | | |
|---|---|---|---|
| Calcd. (%): | C, 64.76; | H, 7.08; | N, 13.13 |
| Found (%): | C, 64.84; | H, 7.30; | N, 12.89 |

IR (KBr) νₘₐₓ (cm⁻¹): 1695, 1654, 1274, 1107
NMR (CDCl₃; 270MHz) δ (ppm): 7.91(1H, d, J=15.8Hz), 7.42(1H, d, J=8.6Hz), 6.98(1H, d, J=15.8Hz), 6.69 (1H, d, J=8.6Hz), 4.11(2H, t, J=7.4Hz), 4.03(3H, s), 4.03-3.95(2H, m), 3.87(3H, s), 3.77(3H, s), 2.19(3H, s), 1.85-1.55(4H, m), 1.03-0.94(6H, m)

### Reference Example 13

### (E)-1,3-Dipropyl-8-(2,3,4-trimethoxystyryl)xanthine (Compound 20)

Substantially the same procedure as in Reference Example 1 was repeated using 2.00 g (8.85 mmol) of 5,6-diamino-1,3-dipropyluracil and 2.32 g (9.73 mmol) of 2,3,4-trimethoxycinnamic acid. Then, the resultant crude crystals were recrystallized from 2-propanol/water to give 1.84 g (yield 49%) of Compound 20 as pale yellow needles.
Melting Point: 246.5-246.8°C

| Elemental Analysis: C₂₂H₂₈N₄O₅ | | | |
|---|---|---|---|
| Calcd. (%): | C, 61.66; | H, 6.58; | N, 13.07 |
| Found (%): | C, 61.50; | H, 6.89; | N, 13.06 |

IR (KBr) νₘₐₓ (cm⁻¹): 1703, 1651, 1504
NMR (CDCl₃; 270MHz) δ (ppm): 12.72(1H, brs), 7.92 (1H, d, J=16.5Hz), 7.31(1H, d, J=8.7Hz), 7.09(1H, d, J=16.5Hz), 6.71(1H, d, J=8.7Hz), 4.25-4.10(4H, m), 3.95(3H, s), 3.91(3H, s), 3.90(3H, s), 2.00-1.65(4H, m), 1.10-0.85(6H, m)

### Reference Example 14

### (E)-7-Methyl-1,3-dipropyl-8-(2,3,4-trimethoxystyryl)xanthine (Compound 21)

Substantially the same procedure as in Reference Example 1 was repeated using 2.50 g (5.84 mmol) of Compound 20 obtained in Reference Example 13 in place of Compound B. Then, the resultant crude crystals were recrystallized from ethanol to give 1.70 g (yield 66%) of Compound 21 as yellow needles.
Melting Point: 153.5-153.8°C

| Elemental Analysis: C₂₃H₃₀N₄O₅ | | | |
|---|---|---|---|
| Calcd. (%): | C, 62.42; | H, 6.83; | N, 12.66 |
| Found (%): | C, 62.77; | H, 7.25; | N, 12.65 |

IR (KBr) νₘₐₓ (cm⁻¹): 1699, 1657, 1590, 1497, 1439
NMR (CDCl₃; 270MHz) δ (ppm): 7.88(1H, d, J=15.8Hz), 7.28(1H, d, J=8.9Hz), 7.02(1H, d, J=15.8Hz), 6.71 (1H, d, J=8.9Hz), 4.25-3.95(4H, m), 4.03(3H, s), 3.97(3H, s), 3.91(3H, s), 3.90(3H, s), 2.00-1.65 (4H, m), 1.10-0.85(6H, m)

### Reference Example 15

### (E)-1,3-Dipropyl-8-(2,4,5-trimethoxystyryl)xanthine (Compound 22)

Substantially the same procedure as in Reference Example 1 was repeated using 2.00 g (8.85 mmol) of 5,6-diamino-1,3-dipropyluracil and 2.32 g (9.73 mmol) of 2,4,5-trimethoxycinnamic acid. Then, the resultant crude crystals were recrystallized from 2-propanol/water to give 870 mg (yield 23%) of Compound 22 as a pale yellow powder.
Melting Point: 254.5-255.7°C

| Elemental Analysis: C₂₂H₂₈N₄O₅ | | | |
|---|---|---|---|
| Calcd. (%): | C, 61.66; | H, 6.58; | N, 13.07 |
| Found (%): | C, 61.94; | H, 6.97; | N, 13.06 |

IR (KBr) νₘₐₓ (cm⁻¹): 1693, 1650, 1517
NMR (CDCl₃; 270MHz) δ (ppm): 12.53(1H, brs), 7.97 (1H, d, J=16.5Hz), 7.10(1H, s), 6.99(1H, d, J=16.5Hz), 6.54(1H, s), 4.25-4.10(4H, m), 3.95(3H, s), 3.90(6H, s), 1.90-1.65(4H, m), 1.01(3H, t, J=7.6Hz), 0.86(3H, t, J=7.6Hz)

### Reference Example 16

### (E)-7-Methyl-1,3-dipropyl-8-(2,4,5-trimethoxystyryl)xanthine (Compound 23)

Substantially the same procedure as in Reference Example 1 was repeated using 0.5 g (1.17 mmol) of Compound 22 obtained in Reference Example 15 in place of Compound B. Then, the resultant crude crystals were recrystallized from toluene/hexane to give 200 mg (yield 39%) of Compound 23 as a pale yellow powder.
Melting Point: 195.5-196.2°C

| Elemental Analysis: C₂₃H₃₀N₄O₅ | | | |
|---|---|---|---|
| Calcd. (%): | C, 62.42; | H, 6.83; | N, 12.66 |
| Found (%): | C, 62.14; | H, 7.12; | N, 12.56 |

IR (KBr) νₘₐₓ (cm⁻¹): 1688, 1653, 1515, 1439, 1214
NMR (CDCl₃; 270MHz) δ (ppm): 7.93(1H, d, J=15.8Hz), 7.05(1H, s), 6.94(1H, d, J=15.8Hz), 6.54(1H, s), 4.15-3.90(4H, m), 4.04(3H, s), 3.95(3H, s), 3.93 (3H, s), 3.91(3H, s), 1.90-1.65(4H, m), 1.03-0.94 (6H, m)

### Reference Example 17

### (E)-8-(2,4-Dimethoxystyryl)-1,3-dipropylxanthine (Compound 24)

Substantially the same procedure as in Reference Example 1 was repeated using 3.0 g (13.3 mmol) of 5,6-diamino-1,3-dipropyluracil and 3.04 g (14.60 mmol) of 2,4-dimethoxycinnamic acid. Then, the resultant crude crystals were recrystallized from dioxane/water to give 1.26 g (yield 24%) of Compound 24 as white crystals.
Melting Point: 273.1-273.7°C

| Elemental Analysis: C₂₁H₂₆N₄O₄ | | | |
|---|---|---|---|
| Calcd. (%): | C, 63.30; | H, 6.57; | N, 14.06 |
| Found (%): | C, 62.94; | H, 6.78; | N, 14.03 |

IR (KBr) νₘₐₓ (cm⁻¹): 1693, 1645, 1506
NMR (DMSO-d₆; 270MHz) δ (ppm): 13.39(1H, brs), 7.78 (1H, d, J=16.5Hz), 7.54(1H, d, J=8.2Hz), 6.95(1H, d, J=16.5Hz), 6.63(1H, d, J=2.3Hz), 6.00(1H, dd, J=8.2, 2.3Hz), 4.01-3.85(4H, m), 3.89(3H, s), 3.82 (3H, s), 1.79-1.50(4H, m), 0.93-0.87(6H, m)

### Reference Example 18

### (E)-8-(2,4-Dimethoxystyryl)-7-methyl-1,3-dipropylxanthine (Compound 25)

Substantially the same procedure as in Reference Example 1 was repeated using 600 mg (1.51 mmol) of Compound 24 obtained in Reference Example 17 in place of Compound B. Then, the resultant crude crystals were recrystallized from hexane/ethyl acetate to give 556 mg (yield 90%) of Compound 25 as brown needles.
Melting Point: 167.6-167.9°C

| Elemental Analysis: C₂₂H₂₈N₄O₄ | | | |
|---|---|---|---|
| Calcd. (%): | C, 64.06; | H, 6.84; | N, 13.58 |
| Found (%): | C, 63.98; | H, 6.94; | N, 13.61 |

IR (KBr) νₘₐₓ (cm⁻¹): 1691, 1653, 1603, 1437
NMR (CDCl₃; 270MHz) δ (ppm): 7.92(1H, d, J=15.8Hz), 7.48(1H, d, J=8.6Hz), 6.98(1H, d, J=15.8Hz), 6.54 (1H, dd, J=8.6, 2.3Hz), 6.50(1H, d, J=2.3Hz), 4.14-3.95(4H, m), 4.02(3H, s), 3.93(3H, s), 3.86 (3H, s), 1.91-1.65(4H, m), 1.03-0.94(6H, m)

### Reference Example 19

### (E)-8-(4-Benzyloxy-3,5-dimethoxystyryl)-1,3-dipropylxanthine (Compound 26)

A mixture of 5.0 g (22.3 mmol) of 4-hydroxy-3,5-dimethoxycinnamic acid, 8.0 ml (66.9 mmol) of benzyl bromide, and potassium carbonate was stirred in 50 ml of dimethylformamide at 70°C for 2 hours. Insoluble matters were filtered off and the filtrate was poured into 500 ml of water. The mixture was extracted three times with 100 ml of chloroform. The extract was washed twice with water and twice with a saturated aqueous solution of sodium chloride, and dried over anhydrous sodium sulfate, followed by evaporation under reduced pressure. To the residue were added 50 ml of an aqueous 2N sodium hydroxide solution and 50 ml of ethanol, followed by heating under reflux for 15 minutes. After cooling, the solution was adjusted to pH 3 with a concentrated hydrochloric acid solution and extracted three times with 50 ml of chloroform. The extract was washed with a saturated aqueous solution of sodium chloride, and dried over anhydrous sodium sulfate, followed by evaporation under reduced pressure. The residue was recrystallized from hexane to give 5.4 g (yield 77%) of (E)-4-benzyloxy-3,5-dimethoxycinnamic acid (Compound G) as pale yellow needles.
Melting Point: 101.8-102.3°C

| Elemental Analysis: C₁₈H₁₈O₅ | | |
|---|---|---|
| Calcd. (%): | C, 68.77; | H, 5.77 |
| Found (%): | C, 68.95; | H, 5.79 |

IR (KBr) νₘₐₓ (cm⁻¹): 2900(br), 1683, 1630, 1579, 1502, 1281, 1129
NMR (CDCl₃; 90MHz) δ (ppm): 7.80(1H, d, J=16Hz), 7.55-7.20(5H, m), 6.80(2H, s), 6.30(1H, d, J=16Hz), 5.08(2H, s)

Substantially the same procedure as in Reference Example 1 was repeated using 3.30 g (14.5 mmol) of 5,6-diamino-1,3-dipropyluracil and 5.0 g (15.9 mmol) of Compound G. Then, the resultant crude crystals were recrystallized from ethanol/2-propanol to give 5.44 g (Yield 74%) of Compound 26 as a white powder.
Melting Point: 221.1-221.4°C

| Elemental Analysis: C₂₈H₃₂N₄O₅ | | | |
|---|---|---|---|
| Calcd. (%): | C, 66.65; | H, 6.39; | N, 11.10 |
| Found (%): | C, 66.65; | H, 6.51; | N, 11.01 |

IR (KBr) νₘₐₓ (cm⁻¹): 1704, 1637, 1582, 1505
NMR (CDCl₃; 90MHz) δ (ppm): 7.69(1H, d, J=16Hz), 7.55-7.20(5H, m), 6.96(1H, d, J=16Hz), 6.80(2H, s), 5.08(2H, s), 4.25-3.95(4H, m), 3.88(6H, s), 2.10-1.65(4H, m), 1.20-0.80(6H, m)

### Reference Example 20

### (E)-8-(4-Benzyloxy-3,5-dimethoxystyryl)-7-methyl-1,3-dipropylxanthine (Compound 27)

Substantially the same procedure as in Reference Example 1 was repeated using 8.20 g (14.5 mmol) of Compound 26 obtained in Reference Example 19 in place of Compound B. Then, the resultant crude crystals were recrystallised from 2-propanol/water to give 4.78 g (yield 64%) of Compound 27 as a white powder.
Melting Point: 164.7-165.1°C

| Elemental Analysis: C₂₉H₃₄N₄O₅ | | | |
|---|---|---|---|
| Calcd. (%): | C, 67.16; | H, 6.60; | N, 10.80 |
| Found (%): | C, 67.01; | H, 6.61; | N, 10.70 |

IR (KBr) νₘₐₓ (cm⁻¹) : 1695, 1659, 1580, 1542, 1505, 1455, 1335
NMR (CDCl₃; 90MHz) δ (ppm): 7.70(1H, d, J=16Hz), 7.55-7.20(5H, m), 6.78(2H, s), 6.72(1H, d, J=16Hz), 5.07(2H, s), 4.25-3.95(4H, m), 4.07(3H, s), 3.89(6H, s), 2.10-1.65(4H, m), 1.20-0.85(6H, m)

### Reference Example 21

### (E)-8-(2,3-Dimethoxystyryl)-1,3-dipropylxanthine (Compound 28)

Substantially the same procedure as in Reference Example 1 was repeated using 2.0 g (8.85 mmol) of 5,6-diamino-1,3-dipropyluracil and 2.2 g (10.6 mmol) of 2,3-dimethoxycinnamic acid. Then, the resultant crude crystals were recrystallized from chloroform/cyclohexane to give 1.26 g (yield 36%) of Compound 28 as yellow crystals.
Melting Point: 236.0-236.5°C

| Elemental Analysis: C₂₁H₂₆N₄O₄ | | | |
|---|---|---|---|
| Calcd. (%): | C, 63.30; | H, 6.57; | N, 14.06 |
| Found (%): | C, 62.99; | H, 6.71; | N, 13.83 |

IR (KBr) νₘₐₓ (cm⁻¹): 1701, 1652, 1271
NMR (DMSO-d₆; 270MHz) δ (ppm): 13.63 (1H, brs), 7.84 (1H, d, J=16.8Hz), 7.28(1H, d, J=6.8Hz), 7.14-7.05 (3H, m), 4.00(2H, t, J=7.3Hz), 3.88-3.78(2H, m), 3.83(3H, s), 3.79(3H, s), 1.80-1.50(4H, m), 0.93-0.85(6H, m)

### Reference Example 22

### (E)-8-(2,3-Dimethoxystyryl)-7-methyl-1,3-dipropylxanthine (Compound 29)

Substantially the same procedure as in Reference Example 1 was repeated using 1.5 g (3.77 mmol) of Compound 28 obtained in Reference Example 21 in place of Compound B. Then, the resultant crude crystals were recrystallized from toluene/cyclohexane to give 1.22 g (yield 79%) of Compound 29 as pale brown needles.
Melting Point: 163.5-163.7°C

| Elemental Analysis: C₂₂H₂₈N₄O₄ | | | |
|---|---|---|---|
| Calcd. (%): | C, 64.06; | H, 6.84; | N, 13.58 |
| Found (%): | C, 64.03; | H, 7.12; | N, 13.42 |

IR (KBr) νₘₐₓ (cm⁻¹): 1695, 1657, 1272
NMR (DMSO-d₆; 270MHz) δ (ppm): 7.88(1H, d, J=15.8Hz), 7.50(1H, dd, J=1.7, 7.6Hz), 7.32(1H, d, J=15.8Hz), 7.17-7.06(2H, m), 4.02(3H, s), 4.02-3.98(2H, m), 3.86-3.81(2H, m), 3.84(3H, s), 3.79(3H, s), 1.80-1.65(2H, m), 1.65-1.50(2H, m), 0.93-0.84(6H, m)

### Reference Example 23

### (E)-8-(3,4-Dimethylstyryl)-1,3-dipropylxanthine (Compound 30)

Substantially the same procedure as in Reference Example 1 was repeated using 5.90 g (26.0 mmol) of 5,6-diamino-1,3-dipropyluracil and 5.5 g (31.3 mmol) of 3,4-dimethylcinnamic acid. Then, the resultant crude crystals were recrystallized from dioxane/water to give 7.70 g (yield 81%) of Compound 30 as a white powder.
Melting Point: 252.7-254.0°C

| Elemental Analysis: C₂₁H₂₆N₄O₂ | | | |
|---|---|---|---|
| Calcd. (%): | C, 68.83; | H, 7.15; | N, 15.29 |
| Found (%): | C, 68.43; | H, 7.22; | N, 15.22 |

IR (KBr) νₘₐₓ (cm⁻¹): 1700, 1648, 1490
NMR (DMSO-d₆; 270MHz) δ (ppm): 7.40(1H, d, J=16.2Hz), 7.37(1H, s), 7.29(1H, d, J=7.2Hz), 7.14(1H, d, J=7.2Hz), 6.95(1H, d, J=16.2Hz), 3.95(2H, t, J=7.2Hz), 3.83(2H, t, J=7.4Hz), 2.25(3H, s), 2.23 (3H, s), 1.80-1.55(4H, m), 1.00-0.90(6H, m)

### Reference Example 24

### (E)-8-(3,4-Dimethylstyryl)-7-methyl-1,3-dipropylxanthine (Compound 31)

Substantially the same procedure as in Reference Example 1 was repeated using 6.50 g (17.8 mmol) of Compound 30 obtained in Reference Example 23 in place of Compound B. Then, the resultant crude crystals were recrystallized from ethanol/water to give 5.62 g (yield 83%) of Compound 31 as white needles.
Melting Point: 169.3-170.3°C

| Elemental Analysis: C₂₂H₂₈N₄O₂ | | | |
|---|---|---|---|
| Calcd. (%): | C, 69.45; | H, 7.42; | N, 14.72 |
| Found (%): | C, 69.33; | H, 7.42; | N, 14.86 |

IR (KBr) νₘₐₓ (cm⁻¹): 1693, 1656
NMR (DMSO-d₆; 270MHz) δ (ppm): 7.59(1H, d, J=15.8Hz), 7.58(1H, s), 7.49(1H, d, J=7.6Hz), 7.26(1H, d, J=15.8Hz), 7.19(1H, d, J=7.6Hz), 4.02(3H, s), 4.05-3.90(2H, m), 3.84(2H, t, J=7.4Hz), 2.27(3H, s), 2.25(3H, s), 1.85-1.50(4H, m), 1.00-0.85(6H, m)

### Reference Example 25

(E)-8-(3,5-Dimethoxystyryl)-1,3-dipropylxanthine (Compound 32)

Substantially the same procedure as in Reference Example 1 was repeated using 3.95 g (17.5 mmol) of 5,6-diamino-1,3-dipropyluracil and 4.0 g (19.2 mmol) of 3,5-dimethoxycinnamic acid. Then, the resultant crude crystals were recrystallized from dimethylformamide/water to give 3.78 g (yield 54%) of Compound 32 as a white powder.
Melting Point: 248.7-250.3°C

| Elemental Analysis: C₂₁H₂₆N₄O₄ | | | |
|---|---|---|---|
| Calcd. (%): | C, 63.30; | H, 6.58; | N, 14.06 |
| Found (%): | C, 63.02; | H, 6.71; | N, 14.06 |

IR (KBr) νₘₐₓ (cm⁻¹): 1687, 1631, 1588, 1494
NMR (DMSO-d₆; 270MHz) δ (ppm): 7.56(1H, d, J=16.6Hz), 7.08(1H, d, J=16.6Hz), 6.78(2H, d, J=2.0Hz), 6.50 (1H, t, J=2.0Hz), 3.98(2H, t, J=7.3Hz), 3.85(2H, t, J=7.3Hz), 3.79(6H, s), 1.80-1.50(4H, m), 0.92-0.84(6H, m)

### Reference Example 26

### (E)-8-(3,5-Dimethoxystyryl)-7-methyl-1,3-dipropylxanthine (Compound 33)

Substantially the same procedure as in Reference Example 1 was repeated using 3.23 g (8.27 mmol) of Compound 32 obtained in Reference Example 25 in place of Compound B. Then, the resultant crude crystals were recrystallized from acetonitrile to give 2.96 g (yield 87%) of Compound 33 as white needles.
Melting Point: 178.0-178.2°C

| Elemental Analysis: C₂₂H₂₈N₄O₄ | | | |
|---|---|---|---|
| Calcd. (%): | C, 64.06; | H, 6.84; | N, 13.58 |
| Found (%): | C, 63.87; | H, 7.11; | N, 13.66 |

IR (KBr) νₘₐₓ (cm⁻¹): 1692, 1657, 1592
NMR (DMSO-d₆; 270MHz) δ (ppm): 7.59(1H, d, J=15.9Hz), 7.35(1H, d, J=15.9Hz), 6.98(2H, d, J=2.9Hz), 6.51 (1H, t, J=2.9Hz), 4.04(3H, s), 4.10-3.95(2H, m), 3.90-3.75(2H, m), 3.80(6H, s), 1.80-1.50(4H, m), 1.00-0.80(6H, m)

### Reference Example 27

### (E)-8-(3-Nitrostyryl)-1,3-dipropylxanthine (Compound 34)

Substantially the same procedure as in Reference Example 1 was repeated using 4.0 g (17.7 mmol) of 5,6-diamino-1,3-dipropyluracil and 3.8 g (19.5 mmol) of 3-nitrocinnamic acid. Then, the resultant crude crystals were recrystallized from toluene to give 3.86 g (yield 57%) of Compound 34 as pale yellow needles.
Melting Point: 256.5-256.8°C

| Elemental Analysis: C₁₉H₂₁N₅O₄·0.25C₆H₅CH₃ | | | |
|---|---|---|---|
| Calcd. (%): | C, 61.32; | H, 5.70; | N, 17.23 |
| Found (%): | C, 61.64; | H, 5.94; | N, 17.29 |

IR (KBr) νₘₐₓ (cm⁻¹): 1701, 1649, 1529, 1355
NMR (DMSO-d₆; 270MHz) δ (ppm): 8.42(1H, s), 8.19(1H, d, J=8.0Hz), 8.12(1H, d, J=7.6Hz), 7.80-7.65(2H, m), 7.25(1H, d, J=16.5Hz), 4.00(2H, t, J=7.2Hz), 3.86(2H, t, J=7.3Hz), 1.80-1.55(4H, m), 1.00-0.80(6H, m)

### Reference Example 28

### (E)-7-Methyl-8-(3-nitrostyryl)-1,3-dipropylxanthine (Compound 35)

Substantially the same procedure as in Reference Example 1 was repeated using 3.20 g (8.36 mmol) of Compound 34 obtained in Reference Example 27 in place of Compound B. Then, the resultant crude crystals were recrystallized from toluene/cyclohexane to give 2.41 g (yield 73%) of Compound 35 as yellow needles.
Melting Point: 218.2-218.4°C

| Elemental Analysis: C₂₀H₂₃N₅O₄ | | | |
|---|---|---|---|
| Calcd. (%): | C, 60.44; | H, 5.83; | N, 17.62 |
| Found (%): | C, 59.94; | H, 5.97; | N, 17.43 |

IR (KBr) νₘₐₓ (cm⁻¹): 1699, 1662, 1521
NMR (DMSO-d₆; 270MHz) δ (ppm): 8.70(1H, m), 8.24(1H, d, J=7.9Hz), 8.19(1H, dd, J=1.6, 7.6Hz), 7.78(1H, d, J=15.9Hz), 7.71(1H, t, J=7.9Hz), 7.61(1H, d, J=15.9Hz), 4.08(3H, s), 4.01(2H, t, J=7.3Hz), 3.85 (2H, t, J=7.3Hz), 1.85-1.55(4H, m), 0.91(3H, t, J=7.5Hz), 0.87(3H, t, J=7.4Hz)

### Reference Example 29

### (E)-8-(3-Fluorostyryl)-1,3-dipropylxanthine (Compound 36)

Substantially the same procedure as in Reference Example 1 was repeated using 3.95 g (17.5 mmol) of 5,6-diamino-1,3-dipropyluracil and 3.19 g (19.2 mmol) of 3-fluorocinnamic acid. Then, the resultant crude crystals were recrystallized from dimethylformamide/water to give 4.67 g (yield 75%) of Compound 36 as a pale yellow powder.
Melting Point: 265.0-265.9°C

| Elemental Analysis: C₁₉H₂₁N₄O₂F | | | |
|---|---|---|---|
| Calcd. (%): | C, 64.03; | H, 5.94; | N, 15.72 |
| Found (%): | C, 64.02; | H, 5.96; | N, 15.46 |

IR (KBr) νₘₐₓ (cm⁻¹): 1701, 1646
NMR (DMSO-d₆; 270MHz) δ (ppm): 7.63(1H, d, J=16.3Hz), 7.53-7.41(3H, m), 7.23-7.15(1H, m), 7.12(1H, d, J=16.3Hz), 3.99(2H, t, J=7.0Hz), 3.86(2H, t, J=7.3Hz), 1.80-1.50(4H, m), 0.93-0.85(6H, m)

### Reference Example 30

### (E)-8-(3-Fluorostyryl)-7-methyl-1,3-dipropylxanthine (Compound 37)

Substantially the same procedure as in Reference Example 1 was repeated using 2.92 g (8.19 mmol) of Compound 36 obtained in Reference Example 29 in place of Compound B. Then, the resultant crude crystals were recrystallized from toluene/cyclohexane to give 2.67 g (yield 88%) of Compound 37 as pale yellow needles.
Melting Point: 161.9-162.0°C

| Elemental Analysis: C₂₀H₂₃N₄O₂F | | | |
|---|---|---|---|
| Calcd. (%): | C, 64.85; | H, 6.26; | N, 15.12 |
| Found (%): | C, 64.61; | H, 6.40; | N, 14.86 |

IR (KBr) νₘₐₓ (cm⁻¹): 1693, 1656, 1544
NMR (DMSO-d₆; 270MHz) δ (ppm): 7.80-7.60(3H, m), 7.50-7.38(2H, m), 7.19(1H, dt, J=2.3, 8.3Hz), 4.04(3H, s), 4.00(2H, t, J=7.3Hz), 3.84(2H, t, J=7.5Hz), 1.80-1.55 (4H, m), 1.00-0.80 (6H, m)

### Reference Example 31

### (E)-8-(3-Chlorostyryl)-1,3-dipropylxanthine (Compound 38)

Substantially the same procedure as in Reference Example 1 was repeated using 3.95 g (17.5 mmol) of 5,6-diamino-1,3-dipropyluracil and 3.51 g (19.2 mmol) of 3-chlorocinnamic acid. Then, the resultant crude crystals were recrystallized from dimethylformamide/water to give 4.44 g (yield 67%) of Compound 38 as pale yellow crystals.
Melting Point: 258.9-259.4°C

| Elemental Analysis: C₁₉H₂₁N₄O₂Cl | | | |
|---|---|---|---|
| Calcd. (%): | C, 61.21; | H, 5.68; | N, 15.03 |
| Found (%): | C, 61.52; | H, 5.73; | N, 14.79 |

IR (KBr) νₘₐₓ (cm⁻¹): 1700, 1644, 1588, 1494
NMR (DMSO-d₆; 270MHz) δ (ppm): 13.7(1H, brs), 7.71-7.52(3H, m), 7.48-7.39(2H, m), 7.12(1H, d, J=16.3Hz), 3.99(2H, t, J=7.0Hz), 3.86(2H, t, J=7.0Hz), 1.80-1.50(4H, m), 0.93-0.84(6H, m)

### Reference Example 32

### (E)-8-(3-Chlorostyryl)-7-methyl-1,3-dipropylxanthine (Compound 39)

Substantially the same procedure as in Reference Example 1 was repeated using 2.85 g (7.66 mmol) of Compound 38 obtained in Reference Example 31 in place of Compound B. Then, the resultant crude crystals were recrystallized from ethanol to give 2.69 g (yield 91%) of Compound 39 as white needles.
Melting Point: 167.7-167.9°C

| Elemental Analysis: C₂₀H₂₃N₄O₂Cl | | | |
|---|---|---|---|
| Calcd. (%): | C, 62.09; | H, 5.99; | N, 14.48 |
| Found (%): | C, 62.00; | H, 6.08; | N, 14.27 |

IR (KBr) νₘₐₓ (cm⁻¹): 1691, 1657, 1543
NMR (DMSO-d₆; 270MHz) δ (ppm): 7.99(1H, s), 7.72 (1H, d, J=6.6Hz), 7.63(1H, d, J=15.8Hz), 7.50-7.30(3H, m), 4.05(3H, s), 4.00(2H, t, J=7.5Hz), 3.84(2H, t, J=7.4Hz), 1.80-1.55(4H, m), 1.00-0.80(6H, m)

### Reference Example 33

### (E)-8-(2-Chlorostyryl)-1,3-dipropylxanthine (Compound 40)

Substantially the same procedure as in Reference Example 1 was repeated using 3.00 g (13.3 mmol) of 5,6-diamino-1,3-dipropyluracil and 2.67 g (14.6 mmol) of 2-chlorocinnamic acid. Then, the resultant crude crystals were recrystallized from toluene to give 3.72 g (yield 82%) of Compound 40 as white needles.
Melting Point: 269.4-269.9°C

| Elemental Analysis: C₁₉H₂₁N₄O₂Cl | | | |
|---|---|---|---|
| Calcd. (%): | C, 61.21; | H, 5.68; | N, 15.03 |
| Found (%): | C, 60.94; | H, 5.69; | N, 14.68 |

IR (KBr) νₘₐₓ (cm⁻¹): 1695, 1645, 1493
NMR (DMSO-d₆; 270MHz) δ (ppm): 8.00-7.80(2H, m), 7.55-7.50(1H, m), 7.45-7.37(2H, m), 7.12(1H, d, J=16.5Hz), 3.99(2H, t, J=7.3Hz), 3.86(2H, t, J=7.4Hz), 1.80-1.55(4H, m), 1.00-0.80(6H, m)

### Reference Example 34

### (E)-8-(2-Chlorostyryl)-7-methyl-1,3-dipropylxanthine (Compound 41)

Substantially the same procedure as in Reference Example 1 was repeated using 2.37 g (6.37 mml) of Compound 40 obtained in Reference Example 33 in place of Compound B. Then, the resultant crude crystals were recrystallized from ethanol/water to give 1.88 g (yield 77%) of Compound 41 as yellow needles.
Melting Point: 159.0-159.9°C

| Elemental Analysis: C₂₀H₂₃N₄O₂Cl | | | |
|---|---|---|---|
| Calcd. (%): | C, 62.09; | H, 5.99; | N, 14.48 |
| Found (%): | C, 61.75; | H, 6.14; | N, 14.45 |

IR (KBr) νₘₐₓ (cm⁻¹): 1696, 1650, 1544
NMR (DMSO-d₆; 270MHz) δ (ppm): 8.10(1H, dd, J=2.3, 7.3Hz), 7.97(1H, d, J=15.5Hz), 7.55-7.50(1H, m), 7.46-7.35(3H, m), 4.05(3H, s), 4.00(2H, t, J=7.3Hz), 3.84 (2H, t, J=7.3Hz), 1.80-1.55 (4H, m), 1.00-0.80(6H, m)

### Reference Example 35

### (E)-8-(2-Fluorostyryl)-1,3-dipropylxanthine (Compound 42)

Substantially the same procedure as in Reference Example 1 was repeated using 3.00 g (13.3 mmol) of 5,6-diamino-1,3-dipropyluracil and 2.43 g (14.6 mmol) of 2-fluorocinnamic acid. Then, the resultant crude crystals were recrystallized from dioxane/water to give 3.23 g (yield 68%) of Compound 42 as white needles.
Melting Point: 258.8-259.2°C

| Elemental Analysis: C₁₉H₂₁N₄O₂F | | | |
|---|---|---|---|
| Calcd. (%): | C, 64.03; | H, 5.94; | N, 15.72 |
| Found (%): | C, 64.01; | H, 6.11; | N, 15.52 |

IR (KBr) νₘₐₓ (cm⁻¹): 1702, 1648
NMR (DMSO-d₆; 270MHz) δ (ppm): 7.85-7.77(2H, m), 7.46-7.32(1H, m), 7.29-7.23(2H, m), 7.16(1H, d, J=16.5Hz), 3.99(2H, t, J=7.1Hz), 3.86(2H, t, J=7.3Hz), 1.80-1.55(4H, m), 1.00-0.80(6H, m)

### Reference Example 36

### (E)-8-(2-Fluorostyryl)-7-methyl-1,3-dipropylxanthine (Compound 43)

Substantially the same procedure as in Reference Example 1 was repeated using 3.50 g (9.83 mmol) of Compound 42 obtained in Reference Example 35 in place of Compound B. Then, the resultant crude crystals were recrystallized from ethanol/water to give 1.23 g (yield 34%) of Compound 43 as white needles.
Melting Point: 155.5-155.9°C

| Elemental Analysis: C₂₀H₂₃N₄O₂F | | | |
|---|---|---|---|
| Calcd. (%): | C, 64.85; | H, 6.26; | N, 15.12 |
| Found (%): | C, 65.00; | H, 6.44; | N, 15.34 |

IR (KBr) νₘₐₓ (cm⁻¹): 1694, 1660
NMR (DMSO-d₆; 270MHz) δ (ppm): 8.02(1H, t, J=8.3Hz), 7.75(1H, d, J=15.5Hz), 7.47-7.40(2H, m), 7.40-7.25(2H, m), 4.03(3H, s), 4.00(2H, t, J=7.4Hz), 3.84(2H, t, J=7.4Hz), 1.80-1.55(4H, m), 1.00-0.80(6H, m)

### Reference Example 37

### (E)-8-(4-Methoxy-2,5-dimethylstyryl)-1,3-dipropylxanthine (Compound 44)

Substantially the same procedure as in Reference Example 1 was repeated using 2.5 g (11.1 mmol) of 5,6-diamino-1,3-dipropyluracil and 2.51 g (12.17 mmol) of 4-methoxy-2,5-dimethylcinnamic acid. Then, the resultant crude crystals were recrystallized from ethanol/water to give 1.98 g (yield 45%) of Compound 44 as white crystals.
Melting Point: 268.0-269.2°C

| Elemental Analysis: C₂₂H₂₈N₄O₃ | | | |
|---|---|---|---|
| Calcd. (%): | C, 66.65; | H, 7.11; | N, 14.13 |
| Found (%): | C, 66.82; | H, 7.34; | N, 14.14 |

IR (KBr) νₘₐₓ (cm⁻¹): 1694, 1644, 1506, 1261
NMR (DMSO-d₆; 270MHz) δ (ppm): 12.95(1H, brs), 7.95 (1H, d, J=15.8Hz), 7.42(1H, s), 6.89(1H, d, J=15.8Hz), 6.66(1H, s), 4.19-4.07(4H, m), 3.86(3H, s), 2.48(3H, s), 2.21(3H, s), 1.91-1.74(4H, m), 1.02(3H, t, J=6.9Hz), 0.93(3H, t, J=6.9Hz)

### Reference Example 38

### (E)-8-(4-Methoxy-2,5-dimethylstyryl)-7-methyl-1,3-dipropylxanthine (Compound 45)

Substantially the same procedure as in Reference Example 1 was repeated using 973 mg (2.45 mmol) of Compound 44 obtained in Reference Example 37 in place of Compound B. Then, the resultant crude crystals were recrystallized from 2-propanol/water to give 966 mg (yield 96%) of Compound 45 as pale yellow needles.
Melting Point: 245.3-246.3°C

| Elemental Analysis: C₂₃H₃₀N₄O₃ | | | |
|---|---|---|---|
| Calcd. (%): | C, 67.30; | H, 7.36; | N, 13.65 |
| Found (%): | C, 67.37; | H, 7.51; | N, 13.69 |

IR (KBr) νₘₐₓ (cm⁻¹): 1690, 1655, 1508, 1261
NMR (DMSO-d₆; 270MHz) δ (ppm): 7.96(1H, d, J=15.8Hz), 7.41(1H, s), 6.70(1H, d, J=15.8Hz), 6.66(1H, s), 4.14-4.09(2H, m), 4.05(3H, s), 4.01-3.95(2H, m), 2.48(3H, s), 2.22(3H, s), 1.91-1.77(2H, m), 1.74-1.63(2H, m), 1.03-0.94(6H, m)

### Reference Example 39

### (Z)-8-(3,4-Dimethoxystyryl)-7-methyl-1,3-dipropylxanthine (Compound 46) (an about 6 : 4 mixture of Compound 46 and Compound 1)

Compound 1 (2.00 g, 4.85 mmol) obtained in Reference Example 1 was dissolved in 180 ml of chloroform, and the solution was irradiated with sunlight for 24 hours. After careful concentration of the reaction mixture, methanol was added thereto and deposited crystals were collected by filtration. The crystals were dried under reduced pressure to give 1.72 g (yield 86%) of a mixture of Compound 46 and Compound 1 as a pale yellow powder (The ratio of Compound 46 to Compound 1 was about 6 : 4 by NMR analysis).
Melting Point: 115.2-119.4°C

| Elemental Analysis: C₂₂H₂₈N₄O₄ | | | |
|---|---|---|---|
| Calcd. (%): | C, 64.06; | H, 6.84; | N, 13.58 |
| Found (%): | C, 64.02; | H, 6.82; | N, 13.46 |

IR (KBr) νₘₐₓ (cm⁻¹): 1695, 1656, 1521
NMR (DMSO-d₆; 270MHz) δ (ppm): 7.60(1x4/10H, d, J=15.8Hz), 7.40(1x4/10H, d, J=2.0Hz), 7.32-7.17 (2x4/10H + 2x6/10H, m), 6.99(1x4/10H, d, J=8.4Hz), 6.94(1x6/10H, d, J=12.7Hz), 6.92(1x6/10H, d, J=8.2Hz), 6.39(1x6/10H, d, J=12.7Hz), 4.02 (3x4/10H, s), 4.10-3.80(4H, m), 3.85(3x4/10H, s), 3.80(3x4/10H, s), 3.77(6x6/10H, s), 3.64(3x6/10H, s), 1.80-1.55(4H, m), 1.00-0.85(6H, m)

### Reference Example 40

### (E)-8-(4-Ethoxystyryl)-1,3-dipropylxanthine (Compound 47)

Substantially the same procedure as in Reference Example 1 was repeated using 3.0 g (13.3 mmol) of 5,6-diamino-1,3-dipropyluracil and 2.80 g (14.6 mmol) of 4-ethoxycinnamic acid. Then, the resultant crude crystals were recrystallized from dioxane to give 3.57 g (yield 70%) of Compound 47 as pale yellow needles.
Melting Point: 261.6-262.0°C

| Elemental Analysis: C₂₁H₂₆N₄O₃ | | | |
|---|---|---|---|
| Calcd. (%): | C, 65.96; | H, 6.85; | N, 14.65 |
| Found (%): | C, 65.93; | H, 7.13; | N, 14.65 |

IR (KBr) νₘₐₓ (cm⁻¹): 1701, 1635, 1516, 1261
NMR (DMSO-d₆; 270MHz) δ (ppm): 13.37(1H, brs), 7.59 (1H, d, J=16.5Hz), 7.55(2H, d, J=8.6Hz), 6.96(2H, d, J=8.6Hz), 6.88(1H, d, J=16.5Hz), 4.07(2H, q, J=6.9Hz), 3.99(2H, t, J=7.3Hz), 3.86(2H, t, J=7.3Hz), 1.73(2H, m), 1.58(2H, m), 1.34(3H, t, J=6.9Hz), 0.90(3H, t, J=7.3Hz); 0.87(3H, t, J=7.3Hz)

### Reference Example 41

### (E)-8-(4-Ethoxystyryl)-7-methyl-1,3-dipropylxanthine (Compound 48)

Substantially the same procedure as in Reference Example 1 was repeated using 2.0 g (5.23 mmol) of Compound 47 obtained in Reference Example 40 in place of Compound B. Then, the resultant crude crystals were recrystallized from hexane/ethyl acetate to give 1.72 g (yield 83%) of Compound 48 as pale green needles.
Melting Point: 174.7-175.0°C

| Elemental Analysis: C₂₂H₂₈N₄O₃ | | | |
|---|---|---|---|
| Calcd. (%): | C, 66.65; | H, 7.11; | N, 14.13 |
| Found (%): | C, 66.60; | H, 7.20; | N, 14.27 |

IR (KBr) νₘₐₓ (cm⁻¹): 1702, 1660, 1515, 1252
NMR (CDCl₃; 270MHz) δ (ppm): 7.74(1H, d, J=15.8Hz), 7.52(2H, d, J=8.6Hz), 6.92(2H, d, J=8.6Hz), 6.76 (1H, d, J=15.8Hz), 4.09(2H, t, J=7.6Hz), 4.08(2H, q, J=6.9Hz), 4.04(3H, s), 3.99(2H, t, J=7.6Hz), 1.44(3H, t, J=6.9Hz), 1.00(3H, t, J=7.6Hz), 0.97 (3H, t, J=7.6Hz)

### Reference Example 42

### (E)-8-(4-Propoxystyryl)-1,3-dipropylxanthine (Compound 49)

Substantially the same procedure as in Reference Example 1 was repeated using 3.0 g (13.3 mmol) of 5,6-diamino-1,3-dipropyluracil and 3.01 g (14.6 mmol) of 4-propoxycinnamic acid. Then, the resultant crude crystals were recrystallized from dioxane/water to give 1.71 g (yield 33%) of Compound 49 as pale brown needles.
Melting Point: 248.3-248.7°C

| Elemental Analysis: C₂₂H₂₈N₄O₃ | | | |
|---|---|---|---|
| Calcd. (%): | C, 66.65; | H, 7.11; | N, 14.13 |
| Found (%): | C, 66.50; | H, 7.48; | N, 14.25 |

IR (KBr) νₘₐₓ (cm⁻¹): 1694, 1649, 1514, 1253
NMR (DMSO-d₆; 270MHz) δ (ppm): 13.34(1H, brs), 7.58 (1H, d, J=16.5Hz), 7.55(2H, d, J=8.6Hz), 6.99(2H, d, J=8.6Hz), 6.88(1H, d, J=16.5Hz), 4.01-3.95(4H, m), 3.86(2H, t, J=7.3Hz), 1.78-1.70(4H, m), 1.62-1.54(2H, m), 0.98(3H, t, J=7.3Hz), 0.90(3H, t, J=7.6Hz), 0.87(3H, t, J=7.6Hz)

### Reference Example 43

### (E)-7-Methyl-8-(4-propoxystyryl)-1,3-dipropylxanthine (Compound 50)

Substantially the same procedure as in Reference Example 1 was repeated using 1.0 g (2.52 mmol) of Compound 49 obtained in Reference Example 42 in place of Compound B. Then, the resultant crude crystals were recrystallized from hexane/ethyl acetate to give 863 mg (yield 83%) of Compound 50 as pale yellow needles.
Melting Point: 172.6-173.5°C

| Elemental Analysis: C₂₃H₃₀N₄O₃ | | | |
|---|---|---|---|
| Calcd. (%): | C, 67.30; | H, 7.36; | N, 13.65 |
| Found (%): | C, 67.15; | H, 7.65; | N, 13.58 |

IR (KBr) νₘₐₓ (cm⁻¹): 1699, 1658, 1514, 1252
NMR (CDCl₃; 270MHz) δ (ppm): 7.74(1H, d, J=15.8Hz), 7.52(2H, d, J=8.9Hz), 6.92(2H, d, J=8.9Hz), 6.76 (1H, d, J=15.8Hz), 4.13-3.94(6H, m), 4.04(3H, s), 1.90-1.62 (6H, m), 1.08-0.94 (9H, m)

### Reference Example 44

### (E)-8-(4-Butoxystyryl)-1,3-dipropylxanthine (Compound 51)

Substantially the same procedure as in Reference Example 1 was repeated using 3.0 g (13.3 mmol) of 5,6-diamino-1,3-dipropyluracil and 3.21 g (14.6 mmol) of 4-butoxycinnamic acid. Then, the resultant crude crystals were recrystallized from dioxane/water to give 3.47 g (yield 64%) of Compound 51 as white needles.
Melting Point: 237.3-238.9°C

| Elemental Analysis: C₂₃H₃₀N₄O₃ | | | |
|---|---|---|---|
| Calcd. (%): | C, 67.30; | H, 7.36; | N, 13.65 |
| Found (%): | C, 67.39; | H, 7.45; | N, 13.59 |

IR (KBr) νₘₐₓ (cm⁻¹): 1697, 1644, 1514, 1257
NMR (DMSO-d₆; 270MHz) δ (ppm): 13.37(1H, brs), 7.58 (1H, d, J=16.2Hz), 7.55 (2H, d, J=8.6Hz), 6.97 (2H, d, J=8.6Hz), 6.88 (1H, d, J=16.2Hz), 4.04-3.96 (4H, m), 3.86(2H, t, J=7.3Hz), 1.80-1.37(8H, m), 0.97-0.84 (9H, m)

### Reference Example 45

### (E)-8-(4-Butoxystyryl)-7-methyl-1,3-dipropylxanthine (Compound 52)

Substantially the same procedure as in Reference Example 1 was repeated using 2.0 g (4.87 mmol) of Compound 51 obtained in Reference Example 44 in place of Compound B. Then, the resultant crude crystals were recrystallized from hexane/ethyl acetate to give 1.56 g (yield 75%) of Compound 52 as pale green needles.
Melting Point: 134.8-135.6°C

| Elemental Analysis: C₂₄H₃₂N₄O₃ | | | |
|---|---|---|---|
| Calcd. (%): | C, 67.90; | H, 7.59; | N, 13.20 |
| Found (%): | C, 68.22; | H, 7.88; | N, 13.49 |

IR (KBr) νₘₐₓ (cm⁻¹): 1696, 1651, 1513, 1247
NMR (CDCl₃; 270MHz) δ (ppm): 7.74(1H, d, J=15.5Hz), 7.52(2H, d, J=8.6Hz), 6.92(2H, d, J=8.6Hz), 6.76 (1H, d, J=15.5Hz), 4.13-3.95(6H, m), 4.04(3H, s), 1.88-1.44 (8H, m), 1.03-0.94 (9H, m)

### Reference Example 46

### (E)-8-(3,4-Dihydroxystyryl)-7-methyl-1,3-dipropylxanthine (Compound 53)

Compound 1 (770 mg, 1.87 mmol) obtained in Reference Example 1 was dissolved in 15 ml of methylene chloride. To the solution was added 5.6 ml (5.6 mmol) of boron tribromide (1.0M methylene chloride solution) under ice cooling in argon atmosphere, and the mixture was stirred overnight at room temperature. Methanol was added thereto and the mixture was separated with chloroform-an aqueous solution of sodium bicarbonate. The organic layer was washed with a saturated aqueous solution of sodium chloride and dried over anhydrous sodium sulfate, followed by evaporation under reduced pressure. The residue was purified by silica gel column chromatography to give 550 mg (yield 77%) of Compound 53 as a yellow solid, which was then triturated with ether to give a yellow powder.
Melting Point: 250.1-251.4°C

| Elemental Analysis: C₂₀H₂₄N₄O₄ | | | |
|---|---|---|---|
| Calcd. (%): | C, 62.49; | H, 6.29; | N, 14.57 |
| Found (%): | C, 62.27; | H, 6.48; | N, 14.74 |

IR (KBr) νₘₐₓ (cm⁻¹): 1680, 1640, 1543, 1306
NMR (DMSO-d₆; 270MHz) δ (ppm): 9.31(1H, brs), 8.95(1H, brs), 7.49(1H, d, J=15.8Hz), 7.15(1H, d, J=2.0Hz), 7.04(1H, dd, J=7.9, 2.0Hz), 6.98(1H, d, J=15.8Hz), 6.78(1H, d, J=7.9Hz), 3.99(2H, t, J=7.6Hz), 3.98 (3H, s), 3.84(2H, t, J=7.4Hz), 1.73(2H, m), 1.57 (2H, m), 0.90(3H, t, J=7.4Hz), 0.87(3H, t, J=7.4Hz)

### Reference Example 47

### (E)-8-(3,4-Diethoxystyryl)-7-methyl-1,3-dipropylxanthine (Compound 54)

Compound 53 (390 mg, 1.01 mmol) obtained in Reference Example 46 was dissolved in 10 ml of dimethylformamide. To the solution were added 0.20 ml (2.50 mmol) of ethyl iodide and 420 mg (3.04 mmol) of potassium carbonate, and the mixture was stirred overnight at room temperature. Water was added thereto to dissolve potassium carbonate and deposited crystals were collected by filtration. The collected crude crystals were recrystallized from hexane/ethyl acetate to give 237 mg (yield 53%) of Compound 54 as pale yellow needles.
Melting Point: 173.8-174.0°C

| Elemental Analysis: C₂₄H₃₂N₄O₄ | | | |
|---|---|---|---|
| Calcd. (%): | C, 65.44; | H, 7.32; | N, 12.72 |
| Found (%): | C, 65.42; | H, 7.48; | N, 12.62 |

IR (KBr) νₘₐₓ (cm⁻¹): 1694, 1653, 1508, 1268
NMR (CDCl₃; 270MHz) δ (ppm): 7.71(1H, d, J=15.5Hz), 7.15(1H, dd, J=8.3, 2.0Hz), 7.10(1H, d, J=2.0Hz), 6.89(1H, d, J=8.3Hz), 6.74(1H, d, J=15.5Hz), 4.16 (2H, q, J=6.9Hz), 4.14(2H, q, J=6.9Hz), 4.08-3.95 (4H, m), 4.05(3H, s), 1.91-1.76(2H, m), 1.76-1.62 (2H, m), 1.49(3H, t, J=6.9Hz), 1.48(3H, t, J=6.9Hz), 1.00(3H, t, J=7.6Hz), 0.97(3H, t, J=7.6Hz)

### Reference Example 48

### (E)-8-(3-Bromo-4-methoxystyryl)-1,3-dipropylxanthine (Compound 55)

Substantially the same procedure as in Reference Example 1 was repeated using 3.0 g (13.3 mmol) of 5,6-diamino-1,3-dipropyluracil and 3.75 g (14.6 mmol) of 3-bromo-4-methoxycinnamic acid. Then, the resultant crude crystals were recrystallized from dioxane to give 3.43 g (yield 58%) of Compound 55 as yellow needles.
Melting Point: 279.8-280.6°C

| Elemental Analysis: C₂₀H₂₃N₄O₃Br | | | |
|---|---|---|---|
| Calcd. (%): | C, 53.70; | H, 5.18; | N, 12.52 |
| Found (%): | C, 53.77; | H, 5.20; | N, 12.49 |

IR (KBr) νₘₐₓ (cm⁻¹): 1685, 1633, 1599, 1503, 1279
NMR (DMSO-d₆; 270MHz) δ (ppm): 13.42(1H, brs), 7.85 (1H, d, J=2.0Hz), 7.61(1H, dd, J=8.4, 2.0Hz), 7.55 (1H, d, J=16.3Hz), 7.15(1H, d, J=8.4Hz), 6.94(1H, d, J=16.3Hz), 3.98(2H, t, J=7.4Hz), 3.89(3H, s), 3.86(2H, t, J=7.4Hz), 1.80-1.52(4H, m), 0.89(6H, q, J=7.4Hz)

### Reference Example 49

### (E)-8-(3-Bromo-4-methoxystyryl)-7-methyl-1,3-dipropylxanthine (Compound 56)

Substantially the same procedure as in Reference Example 1 was repeated using 750 mg (1.68 mmol) of Compound 55 obtained in Reference Example 48 in place of Compound B. Then, the resultant crude crystals were recrystallized from hexane/ethyl acetate to give 588 mg (yield 76%) of Compound 56 as pale yellow needles.
Melting Point: 209.4-210.8°C

| Elemental Analysis: C₂₁H₂₅N₄O₃Br | | | |
|---|---|---|---|
| Calcd. (%): | C, 54.67; | H, 5.46; | N, 12.14 |
| Found (%): | C, 54.47; | H, 5.51; | N, 11.91 |

IR (KBr) νₘₐₓ (cm⁻¹): 1693, 1656, 1542, 1500, 1264
NMR (CDCl₃; 270MHz) δ (ppm): 7.83(1H, d, J=2.0Hz), 7.68(1H, d, J=15.8Hz), 7.48(1H, dd, J=8.4, 2.0Hz), 6.92(1H, d, J=8.4Hz), 6.78(1H, d, J=15.8Hz), 4.13-4.07(2H, m), 4.06(3H, s), 4.01-3.97(2H, m), 3.95 (3H, s), 1.90-1.65(4H, m), 1.00(3H, t, J=7.4Hz), 0.97(3H, t, J=7.4Hz)

### Reference Example 50

### (E)-8-(2-Bromo-4,5-dimethoxystyryl)-1,3-dipropylxanthine (Compound 57)

Substantially the same procedure as in Reference Example 1 was repeated using 2.0 g (8.85 mmol) of 5,6-diamino-1,3-dipropyluracil and 2.80 g (9.75 mmol) of 2-bromo-4,5-dimethoxycinnamic acid. Then, the resultant crude crystals were recrystallized from dioxane to give 2.38 g (yield 56%) of Compound 57 as pale yellow needles.
Melting Point: 248.2-249.5°C

| Elemental Analysis: C₂₁H₂₅N₄O₄Br | | | |
|---|---|---|---|
| Calcd. (%): | C, 52.84; | H, 5.28; | N, 11.74 |
| Found (%): | C, 52.73; | H, 5.31; | N, 11.45 |

IR (KBr) νₘₐₓ (cm⁻¹): 1697, 1643, 1506, 1263
NMR (DMSO-d₆; 270MHz) δ (ppm): 13.75(1H, brs), 7.81 (1H, d, J=16.3Hz), 7.39(1H, s), 7.20(1H, s), 7.09 (1H, d, J=16.3Hz), 4.00-3.82(4H, m), 3.86(3H, s), 3.82(3H, s), 1.76-1.54(4H, m), 0.92-0.85(6H, m)

### Reference Example 51

### (E)-8-(2-Bromo-4,5-dimethoxystyryl)-7-methyl-1,3-dipropylxanthine (Compound 58)

Substantially the same procedure as in Reference Example 1 was repeated using 800 mg (1.68 mmol) of Compound 57 obtained in Reference Example 50 in place of Compound B. Then, the resultant crude crystals were recrystallized from dioxane to give 766 mg (yield 93%) of Compound 58 as yellow needles.
Melting Point: 228.8-229.4°C

| Elemental Analysis: C₂₂H₂₇N₄O₄Br | | | |
|---|---|---|---|
| Calcd. (%): | C, 53.78; | H, 5.54; | N, 11.40 |
| Found (%): | C, 53.76; | H, 5.67; | N, 11.16 |

IR (KBr) νₘₐₓ (cm⁻¹): 1688, 1650, 1509, 1266
NMR (CDCl₃; 270MHz) δ (ppm): 8.01(1H, d, J=15.8Hz), 7.11(1H, s), 7.09(1H, s), 6.75(1H, d, J=15.8Hz), 4.15-3.92(4H, m), 4.08(3H, s), 3.95(3H, s), 3.92 (3H, s), 1.91-1.77(2H, m), 1.74-1.63(2H, m), 1.03-0.94 (6H, m)

### Reference Example 52

### (E)-8-(3-Bromo-4,5-dimethoxystyryl)-1,3-dipropylxanthine (Compound 59)

Substantially the same procedure as in Reference Example 1 was repeated using 1.5 g (6.64 mmol) of 5,6-diamino-1,3-dipropyluracil and 2.10 g (7.31 mmol) of 3-bromo-4,5-dimethoxycinnamic acid. Then, the resultant crude crystals were recrystallized from dioxane/water to give 2.11 g (yield 67%) of Compound 59 as white needles.
Melting Point: 276.7-277.5°C

| Elemental Analysis: C₂₁H₂₅N₄O₄Br | | | |
|---|---|---|---|
| Calcd. (%): | C, 52.84; | H, 5.28; | N, 11.74 |
| Found (%): | C, 52.72; | H, 5.16; | N, 11.56 |

IR (KBr) νₘₐₓ (cm⁻¹): 1701, 1650, 1562, 1498
NMR (DMSO-d₆; 270MHz) δ (ppm): 13.44(1H, brs), 7.55 (1H, d, J=16.3Hz), 7.39(1H, d, J=2.0Hz), 7.36(1H, d, J=2.0Hz), 7.07(1H, d, J=16.3Hz), 3.99(2H, t, J=7.4Hz), 3.91(3H, s), 3.86(2H, t, J=7.4Hz), 3.78 (3H, s), 1.77-1.52(4H, m), 0.93-0.85(6H, m)

### Reference Example 53

### (E)-8-(3-Bromo-4,5-dimethoxystyryl)-7-methyl-1,3-dipropylxanthine (Compound 60)

Substantially the same procedure as in Reference Example 1 was repeated using 1.0 g (2.10 mmol) of Compound 59 obtained in Reference Example 52 in place of Compound B. Then, the resultant crude crystals were recrystallized from hexane/ethyl acetate to give 952 mg (yield 93%) of Compound 60 as pale yellow needles.
- Melting Point:: 180.9-181.6°C
- MS-EI m/e:: 490, 492
- IR (KBr) νₘₐₓ (cm⁻¹):: 1691, 1648, 1542, 1493
- NMR (CDCl₃; 270MHz) δ (ppm):: 7.68(1H, d, J=15.8Hz), 7.42(1H, d, J=2.0Hz), 7.02(1H, d, J=2.0Hz), 6.80 (1H, d, J=15.8Hz), 4.13-3.95(4H, m), 4.08(3H, s), 3.94(3H, s), 3.90(3H, s), 1.90-1.65(4H, m), 1.01 (3H, t, J=7.4Hz), 0.97(3H, t, J=7.4Hz)

### Reference Example 54

### (E)-8-(3-Hydroxy-4-methoxystyryl)-7-methyl-1,3-dipropylxanthine (Compound 63)

Compound 53 (500 mg, 1.30 mmol) obtained in Reference Example 46 was dissolved in 10 ml of dimethylformamide. To the solution were added 0.40 ml (6.43 mmol) of methyl iodide and 400 mg (6.50 mmol) of lithium carbonate, and the mixture was stirred at 80°C for 5 hours. Water was added thereto to dissolve lithium carbonate and deposited crystals were collected by filtration. The collected crude crystals were dissolved in chloroform, washed with a saturated aqueous solution of sodium chloride and dried over anhydrous sodium sulfate, followed by evaporation under reduced pressure. The residue was purified by silica gel column chromatography (eluent: chloroform) to give 162 mg (yield 31%) of Compound 63 as yellow grains.
- Melting Point:: 200.3-203.6°C
- IR (KBr) νₘₐₓ (cm⁻¹):: 1683, 1642, 1512, 1278
- NMR (DMSO-d₆; 270MHz) δ (ppm):: 8.98(1H, brs), 7.52(1H, d, J=15.5Hz), 7.22(1H, d, J=2.0Hz), 7.15(1H, dd, J=8.3, 2.0Hz), 7.06(1H, d, J=15.5Hz), 6.96 (1H, d, J=8.3Hz), 4.02-3.97(2H, m), 4.00(3H, s), 3.84-3.82 (2H, m), 3.82(3H, s), 1.80-1.50 (4H, m), 0.90(3H, t, J=7.3Hz), 0.87(3H, t, J=7.3Hz)

## Claims

1. The use of a xanthine derivative of the Formula (I), or a pharmaceutically acceptable salt thereof, in the manufacture of pharmaceutical preparations for use in the treatment of Parkinson's diseases : 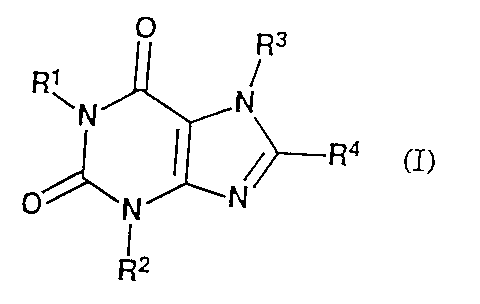 where R¹, R² and R³ are each H, C₁-C₆ alkyl or allyl; and R⁴ is a 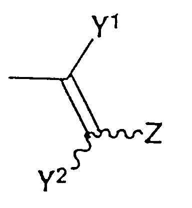 group, where Y¹ and Y² are each H or CH₃ and Z is an aryl group of 6 to 10 carbons or a heterocyclic group,
optionally being substituted by up to 3 substituent(s) selected from C₁-C₆ alkyl, hydroxy, C₁-C₆ alkoxy, halogen, nitro and amino.

2. The use according to claim 1, of compounds of formula (I), where Y¹ and Y² are both H.

3. The use according to claim 1, of compounds of formula (I), where Y¹ and Y² are both H and Z represents a substituted or unsubstituted aryl group, preferably substituted or unsubstituted phenyl.

4. The use according to claim 1, of compounds of formula (I), where Y¹ and Y² are both H and Z is as defined in claim 3 and R³ is C₁-C₆ alkyl, preferably methyl, and where, preferably R¹ and R² are each C₁-C₆ alkyl or allyl.

5. The use according to claim 1, of compounds of formula (I), where R¹ and R² are each C₁-C₆ alkyl or allyl, preferably allyl, methyl, ethyl or propyl, R³ is methyl, Y¹ and Y² are both H and Z represents a substituted phenyl group containing from 1 to 3 C₁-C₆ alkyl or C₁-C₆ alkoxy substituents, preferably methyl, methoxy or ethoxy.

6. The use according to claim 1, of compounds of formula (I), where R¹, R², R³ and Y¹, Y² and Z are as defined in claim 5, and where the configuration at position 8 of the xanthine ring is the (E) form.

7. A compound of the formula (I-a): 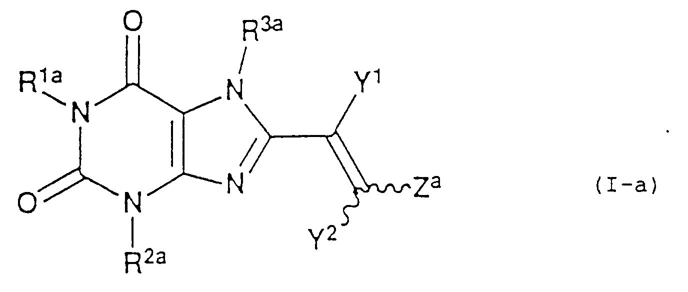 where R^{1a} and R^{2a} are each H, propyl, butyl or allyl;
R^{3a} is H, C₁-C₆ alkyl or allyl:
Z^{a} is naphthyl, optionally containing from 1 to 3 substituent(s) selected from C₁-C₆ alkyl, hydroxy, C₁-C₆ alkoxy, halogen, nitro and amino, or a 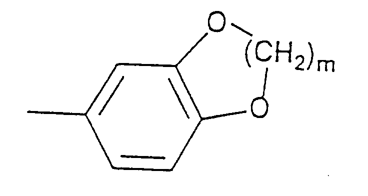 group, where m is 1, 2 or 3; and
Y¹ and Y² are each H or CH₃; or a
pharmaceutically acceptable salt thereof.

8. A compound or salt according to claim 7, where, in said formula (I-a), Z^{a} is a 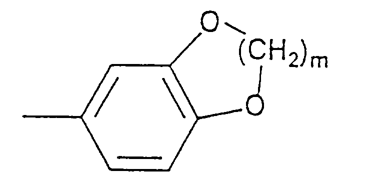 group where m is 1, 2 or 3; R^{3a} is CH₃ and R^{1a} and R^{2a} are both propyl.

9. A compound or salt according to claim 8, where m is 2.

## Patentansprüche

1. Verwendung eines Xanthin-Derivats der Forrnel ( I ) oder eines pharmazeutisch verträglichen Salzes davon, zur Herstellung von Arzneimitteln zur Verwendung bei der Behandlung der Parkinsonschen Krankheit: 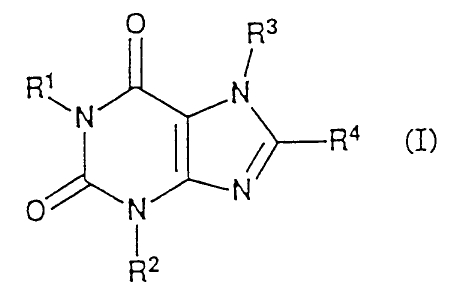 wobei R¹, R² und R³ jeweils Wasserstoffatome, C₁-C₆-Alkylreste oder Allylgruppen sind; und R⁴ ein Rest 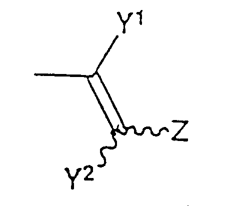 ist, wobei Y¹ und Y² jeweils Wasserstoffatome oder CH₃-Gruppen sind und Z ein Arylrest mit 6 bis 10 Kohlenstoffatomen oder ein heterocyclischer Rest ist, wobei diese gegebenenfalls mit bis zu 3 Substituenten, ausgewählt aus C₁-C₆-Alkylresten, Hydroxylgruppen, C₁-C₆-Alkoxyresten, Halogenatomen, Nitro- und Aminogruppen, substituiert sind.

2. Verwendung nach Anspruch 1, wobei in den Verbindungen der Formel ( I ) Y¹ und Y² jeweils Wasserstoffatome sind.

3. Verwendung nach Anspruch 1, wobei in den Verbindungen der Formel ( I ) Y¹ und Y² jeweils Wasserstoffatome sind und Z einen substituierten oder unsubstituierten Arylrest, bevorzugt eine substituierte oder unsubstituierte Phenylgruppe bedeutet.

4. Verwendung nach Anspruch 1, wobei in den Verbindungen der Formel ( I ) Y¹ und Y² jeweils Wasserstoffatome sind und Z wie in Anspruch 3 definiert ist und R³ ein C₁-C₆-Alkylrest, bevorzugt eine Methylgruppe ist und wobei R¹ und R² bevorzugt C₁-C₆-Alkylreste oder Allylgruppen sind.

5. Verwendung nach Anspruch 1, wobei in den Verbindungen der Formel ( I ) R¹ und R² jeweils C₁-C₆-Alkylreste oder Allylgruppen, bevorzugt Allyl-, Methyl-, Ethyl- oder Propylgruppen sind, R³ eine Methylgruppe ist, Y¹ und Y² jeweils Wasserstoffatome sind und Z eine substituierte Phenylgruppe ist, die 1 bis 3 C₁-C₆-Alkyl- oder C₁-C₆-Alkoxysubstituenten, bevorzugt Methyl-, Methoxy- oder Ethoxygruppen enthält.

6. Verwendung nach Anspruch 1, wobei in den Verbindungen der Formel ( I ) R¹, R², R³ und Y¹, Y² und Z wie in Anspruch 5 definiert sind und wobei die Konfiguration an der 8-Stellung des Xanthinrings die ( E )-Form ist.

7. Verbindung der Formel ( I-a ): 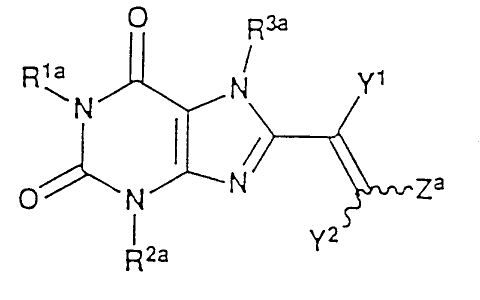 wobei R^{1a} und R^{2a} jeweils Wasserstoffatome, Propyl-, Butyl- oder Allylgruppen sind;
R^{3a} ein Wasserstoffatom, ein C₁-C₆-Alkylrest oder eine Allylgruppe ist;
Z^{a} eine Naphthylgruppe ist, die gegebenenfalls 1 bis 3 Substituenten enthält, ausgewählt aus C₁-C₆-Alkylresten, Hydroxylgruppen, C₁-C₆-Alkoxyresten, Halogenatomen, Nitro- und Aminogruppen, oder eine Gruppe 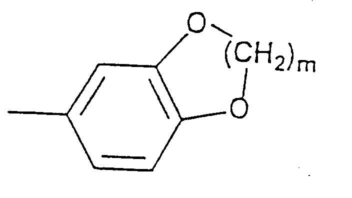 ist,
wobei m die Bedeutung von 1, 2 oder 3 hat; und
Y¹ und Y² jeweils Wasserstoffatome oder CH₃-Gruppen sind; oder ein
pharmazeutisch verträgliches Salz davon.

8. Verbindung oder Salz nach Anspruch 7, wobei in Formel ( I-a ) Z^{a} eine Gruppe 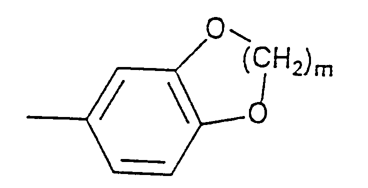 ist, wobei m die Bedeutung von 1, 2 oder 3 hat; R^{3a} eine CH₃-Gruppe ist und R^{1a} und R^{2a} jeweils Propylgruppen sind.

9. Verbindung oder Salz nach Anspruch 8, wobei m die Bedeutung von 2 hat.

## Revendications

1. Emploi, dans la fabrication de préparations pharmaceutiques destinées a être utilisées dans le traitement de la maladie de Parkinson, d'un dérive de xanthine de formule (I) ou d'un sel d'un tel dérivé, admissible en pharmacie : 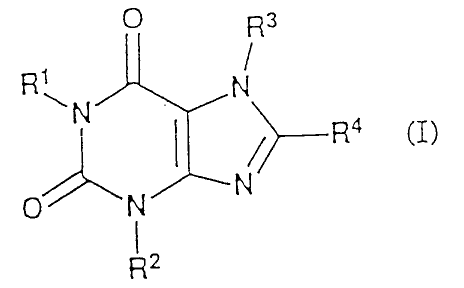 où R¹, R² et R³ représentent chacun un atome d'hydrogène, un groupe alkyle en C₁₋₆ ou un groupe allyle, et R⁴ représente un groupe de formule 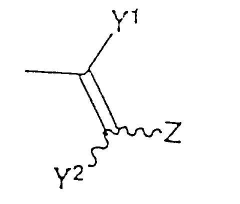 dans laquelle Y¹ et Y² représentent chacun un atome d'hydrogène ou un groupe méthyle, et Z représente un groupe aryle comportant de 6 à 10 atomes de carbone ou un groupe hétérocyclique, portant éventuellement jusqu'à 3 substituants choisis parmi les atomes d'halogène et les groupes alkyle en C₁₋₆, hydroxy, alcoxy en C₁₋₆, nitro et amino.

2. Emploi, conforme à la revendication 1, de composés de formule (I) où Y¹ et Y² représentent tous les deux des atomes d'hydrogène.

3. Emploi, conforme à la revendication 1, de composés de formule (I) où Y¹ et Y² représentent tous les deux des atomes d'hydrogène et Z représente un groupe aryle portant ou non des substituants, et de préférence un groupe phényle portant ou non des substituants.

4. Emploi, conforme à la revendication 1, de composés de formule (I) où Y¹ et Y² représentent tous les deux des atomes d'hydrogène et Z est défini comme dans la revendication 3, R³ représente un groupe alkyle en C₁₋₆, de préférence un groupe méthyle, et de préférence, R¹ et R² représentent chacun un groupe alkyle en C₁₋₆ ou un groupe allyle.

5. Emploi, conforme à la revendication 1, de composés de formule (I) où R¹ et R² représentent chacun un groupe alkyle en C₁₋₆ ou un groupe allyle, et de préférence un groupe allyle, méthyle, éthyle ou propyle, R³ représente un groupe méthyle, Y¹ et Y² représentent tous les deux des atomes d'hydrogène, et Z représente un groupe phényle portant de 1 à 3 substituants alkyle en C₁₋₆ ou alcoxy en C₁₋₆, et de préférence des substituants méthyle, méthoxy ou éthoxy.

6. Emploi, conforme à la revendication 1, de composés de formule (I) où les symboles R¹, R², R³, Y¹, Y² et Z ont les significations indiquées dans la revendication 5 et où le substituant placé en position n° 8 du cycle de xanthine adopte la configuration E.

7. Composé de formule (I-a) : 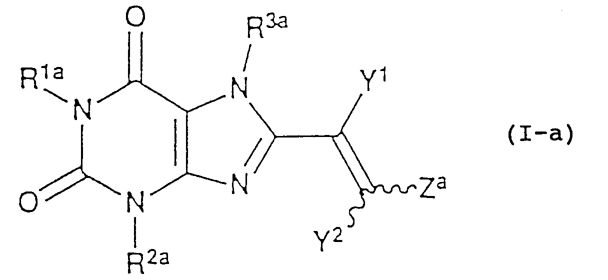 dans laquelle
R^{1a} et R^{2a} représentent chacun un atome d'hydrogène ou un groupe propyle, butyle ou allyle,
R^{3a} représente un atome d'hydrogène, un groupe alkyle en C₁₋₆ ou un groupe allyle,
Z^{a} représente un groupe naphtyle, portant éventuellement de 1 à 3 substituants choisis parmi les atomes d'halogène et les groupes alkyle en C₁₋₆, hydroxy, alcoxy en C₁₋₆, nitro et amino, ou bien un groupe de formule 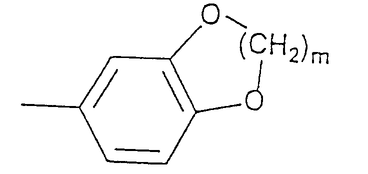
où m vaut 1, 2 ou 3, et
Y¹ et Y² représentent chacun un atome d'hydrogène ou un groupe méthyle,
ou sel admissible en pharmacie d'un tel compose.

8. Composé ou sel conforme à la revendication 7, dans lequel, dans la formule (I-a), R^{1a} et R^{2a} représentent tous les deux des groupes propyle, R^{3a} représente un groupe méthyle et Z^{a} représente un groupe de formule 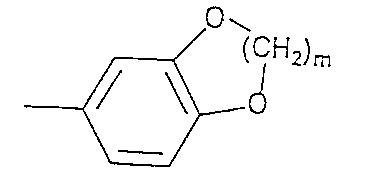 où m vaut 1, 2 ou 3.

9. Composé ou sel conforme à la revendication 8, dans lequel m vaut 2.
